# FASCICULE DE BREVET EUROPEEN

(11) **EP 1 343 814 B1**
(45) Date de publication et mention de la délivrance du brevet: **16.09.2009**
(21) Numéro de dépôt: 01991884.6
(22) Date de dépôt: 20.12.2001
(51) Int. Cl.: C07K 14/35, C07K 9/00, C07H 5/02, A61K 39/04

(54) **GLYCOPEPTIDES IMMUNOGENES, CRIBLAGE, PREPARATION ET APPLICATIONS.**
IMMUNOGENE GLYCOPEPTIDE. SCREENING- UND HERSTELLUNGSVERFAHREN SOWIE VERWENDUNGEN
IMMUNOGENIC GLYCOPEPTIDES, SCREENING, PREPARATION AND USES

(30) Priorité: 21.12.2000 FR 0016808
(43) Date de publication de la demande: 17.09.2003
(73) Titulaire: INSTITUT PASTEUR, 75724 Paris Cédex 15 (FR)
(72) Inventeur: MARCHAL, Gilles, F-94200 IVRY-SUR-SEINE (FR); ROMAIN, Félix, F-91640 FONTENAY-LES-BRIIS (FR); PESCHER, Pascale, F-75018 PARIS (FR)
(74) Mandataire: Vialle-Presles, Marie José
(86) Numéro de dépôt international: PCT/FR2001/004100
(87) Numéro de publication internationale: WO 2002/050108

(56) Documents cités:
- WO-A-00/52046
- WO-A-96/23885
- WO-A-98/16646
- WO-A-98/29132
- DOBOS KAREN M ET AL: "DEFINITION OF THE FULL EXTENT OF GLYCOSYLATION OF THE 45-KILODALTON GLYCOPROTEIN OF MYCOBACTERIUM TUBERCULOSIS." JOURNAL OF BACTERIOLOGY, vol. 178, no. 9, 1996, pages 2498-2506, XP002180487 ISSN: 0021-9193 cité dans la demande
- FRANZYK HENRIK ET AL.: "SYNTHESIS OF ALIPHATIC O-DIMANNOSYL AMINO ACID BUILDING BLOCKS FOR SOLID-PHASE ASSEMBLY OF GLYCOPEPTIDE LIBRARIES" J CHEM SOC PERKIN TRANS 1, 1995, pages 2883-2898, XP002180489 cité dans la demande
- ROMAIN FELIX ET AL: "DEGLYCOSYLATION OF THE 45/47-KILODALTON ANTIGEN COMPLEX OF MYCOBACTERIUM TUBERCULOSIS DECREASES ITS CAPACITY TO ELICIT IN VIVO OR IN VITRO CELLULAR IMMUNE RESPONSES." INFECTION AND IMMUNITY, vol. 67, no. 11, novembre 1999 (1999-11), pages 5567-5572, XP002180488 ISSN: 0019-9567 cité dans la demande

## Description

La présente invention est relative à des glycopeptides immunogènes issus de *Mycobacterium tuberculosis (M. tuberculosis),* utiles pour la vaccination et le diagnostic d'infections dues à ce microorganisme pathogène, ainsi qu'à leurs procédés de sélection et de préparation.

L'infection par *M. tuberculosis* est l'une des plus graves infections en médecine humaine. En effet, 5 à 10 % des personnes infectées par *M. tuberculosis* ayant une réponse immunitaire normale développent une maladie grave (tuberculose) ; cette fréquence est encore plus élevée chez les personnes ayant un déficit de leur réponse immunitaire (infection par le VIH, traitement par des immunodépresseurs, etc...).

Les moyens mis en oeuvre pour prévenir et traiter cette infection comprennent d'une part le dépistage permettant le suivi et le traitement de l'infection et d'autre part la vaccination.

### Diagnostic

Parmi les différentes techniques actuellement disponibles, on peut citer:
- l'obtention de cultures pures de *M. tuberculosis,* qui est le moyen le plus rigoureux de faire le diagnostic de certitude de la tuberculose. C'est une technique moyennement sensible qui permet le diagnostic pour les 2/3 des cas de tuberculose pulmonaire. Les résultats ne sont disponibles qu'après un délai minimum de 3-4 semaines, quelquefois seulement après 2 mois de culture. L'utilisation de techniques de culture employant des précurseurs marqués permet de raccourcir ces délais qui restent cependant importants. Cette mise en évidence de *M. tuberculosis* par culture nécessite un prélèvement contenant des bacilles, parfois difficile à obtenir même pour la tuberculose pulmonaire où environ 1/3 des cas ne reçoit pas de confirmation biologique. Quelquefois cet examen nécessite une intervention médicale spécialisée (ponction lombaire du liquide céphalo-rachidien ou biopsie ganglionnaire) pour des formes extra-pulmonaires de la maladie.
- les techniques microbiologiques basées sur la génétique moléculaire (PCR) se heurtent à la même nécessité d'obtenir un prélèvement contenant des bactéries. Par ailleurs, du fait de la présence dans le prélèvement d'inhibiteurs de la réaction PCR dont l'origine est impossible à contrôler, ces techniques sont parfois inutilisables. Leur validation en pratique courante n'a pas été obtenue.
- il n'existe pas à l'heure actuelle de sérodiagnostic qui possède une sensibilité et une spécificité compatibles avec un usage diagnostique.
- la réaction à la tuberculine montre qu'un sujet est sensibilisé, a été infecté par *M. tuberculosis* ou a été vacciné par le BCG. La tuberculine est en effet un mélange d'antigènes de *M. tuberculosis* et est donc incapable de faire le diagnostic entre une infection par *M. tuberculosis* et la vaccination par le BCG du fait de réactions croisées très nombreuses entre les antigènes du vaccin et *M. tuberculosis.* D'autre part, cette réaction à la tuberculine ne permet pas de distinguer une tuberculose, maladie active, d'une infection par *M. tuberculosis.*

### Vaccin

La vaccination par le BCG permet de contrôler l'infection primaire (multiplication initiale de *M. tuberculosis*) mais surtout la dissémination secondaire de ces bacilles. Elle contribue vraisemblablement à diminuer l'incidence des infections latentes contre lesquelles aucun traitement efficace n'est disponible actuellement. Le BCG a été utilisé pour vacciner plus de 3 milliards d'individus contre la tuberculose, sans effets secondaires particuliers. Lors de la vaccination par le BCG il y a une multiplication locale de ces bacilles de virulence atténuée. Une immunité cellulaire est induite. Elle se traduit par une hypersensibilité de type retardé (HSR) dirigée contre les protéines, antigènes, de mycobactéries, c'est la réaction à la tuberculine, et par une résistance accrue à l'infection par *M. tuberculosis.* Ces deux réponses immunitaires (sensibilisation de type HSR et résistance accrue) ont pour supports des lymphocytes T réagissant avec des antigènes de mycobactéries.

Le BCG protège bien contre les formes aiguës de l'infection (méningite tuberculeuse de l'enfant, par exemple). Son efficacité est plus variable chez l'adulte. L'existence d'une réactivité croisée entre le BCG et d'autres mycobactéries n'appartenant pas au complexe *tuberculosis* ainsi que l'absence, dans le génome de BCG, de certains antigènes immunogènes de *Mycobacterium tuberculosis* ou un profil d'expression différent de ces antigènes au cours de l'infection pourraient expliquer l'efficacité variable du BCG.

En outre, le BCG est une souche vivante de virulence atténuée. Celle-ci possède donc un pouvoir pathogène résiduel qui en interdit l'utilisation chez les individus immunodéprimés, notamment chez les sujets reconnus pour être infectés par le virus de l'immunodéficience humaine (VIH).

Pour lutter plus efficacement contre cette infection, il serait judicieux de disposer d'outils diagnostiques et de vaccins, notamment d'un vaccin "sous-unité", et donc sans danger, à base d'antigènes protecteurs du microorganisme pathogène responsable de cette infection.

Un certain nombre d'études ont été faites dans ce sens afin de trouver la ou les molécules de ce microorganisme pathogène, susceptible(s) d'induire une forte réponse immunitaire protectrice. Ainsi, J. Hess et al. (C.R. Acad. Sci. Paris, 1999, 322: 953-958) ont fait le point sur les propriétés que devraient avoir des antigènes aptes à être utilisés comme vaccin contre la tuberculose. Dans cette revue, ils soulignent l'importance d'utiliser une combinaison d'antigènes préalablement sélectionnés plutôt qu'un antigène unique. Ils recommandent notamment de sélectionner ces antigènes sur la base de critères tels que la présence de zones hautement conservées parmi les différentes souches, les différences dans le profil d'expression des gènes des souches virulentes et des souches atténuées, la réactivité vis-à-vis des cellules effectrices de la réponse immunitaire (lymphocytes B, T CD4⁺, T CD8⁺), la capacité de ces antigènes à se lier à une majorité des molécules HLA du complexe majeur d'histocompatibilité (CMH).

Certains de ces antigènes sont présents sous forme d'antigènes sécrétés: MPT59 (30 kDa), 85A (32 kDa), MPT64 (23 kDa), hsp71 (71 kfla), MPT51 (24 kDa), MPT63 (16 kDa) et ESAT-6 (6 kDa), (Andersen, Infect. Immun, 1994, 62, 2536-2544*;* Horwitz et aL., PNAS, 1995, 92, 1530-1534)*.* Ces antigènes de *M. tuberculosis* ont déjà été proposés comme des candidats potentiels d'une composition vaccinale car reconnus préférentiellement par des lymphocytes T CD4⁺ (Andersen, et al., précité; Horwitz et al., précité).

Il a également été proposé d'isoler, à partir des antigènes de *M tuberculosis,* des peptides contenant des épitopes capables d'être présentés par une molécule de classe Il du CMH et d'être reconnus par les lymphocytes T CD4+ spécifiques ; de tels épitopes ont notamment été rapportés pour deux protéines : ESAT-6 (Olsen et al., Eur. J. Immunol., 2000, 30, 1724-1732) et MPT-39 (Mustafa et al., Inf Immunol., 2000, 68, 3933-3940).

Plusieurs observations ont été précédemment faites par les Inventeurs (Romain et al., Inf. Immun, 1993, 61, 742-750 ; Romain et al. Proc. Natl. Acad. Sci. USA 1993, 90: 5322-5326):
- seules les bactéries vivantes sont susceptibles d'induire une immunité protectrice, les bactéries tuées induisant aussi une réponse immunitaire, mais sans protection;
- il existe dans le milieu de culture des protéines libérées par les bactéries au cours de leur croissance et susceptibles d'être reconnues par le système immunitaire d'animaux vaccinés avec des bactéries vivantes, protéines qui ne sont pas ou très peu reconnues après immunisation par des bactéries tuées.

Grâce à ce double critère de sélection, deux protéines nouvelles ont été purifiées. Une protéine sécrétée par *M. tuberculosis,* dénommée Apa, ou MPT-32 ou complexe antigénique de 45/47 kDa, est le produit du gène Rv 1860 (Laqueyrerie et al. Infect. Immun. 1995, 63: 4003-4010). La seconde molécule est un peptide interne d'une sérine protéase putative codée par le gène Rv1796.

En utilisant la protéine Apa native comme antigène, les Inventeurs ont précédemment montré que cette protéine qui ne représente que 2 % des protéines sécrétées par les bacilles du groupe de la tuberculose *(M. tuberculosis, M. bovis et BCG)* en culture est un antigène immunodominant, reconnu de façon très efficace par les lymphocytes T CD4+ spécifiques provenant d'animaux infectés par *M. tuberculosis* ou vaccinés par le BCG (Romain et al., Inf Immun, 1999, 67, 5567-5572 *;* Horn et al., J. Biol. Chem., 1999, 274, 32023-32030).

Dans ces mêmes travaux, les Inventeurs ont également montré que la mannosylation de l'Apa était essentielle pour l'activité antigénique de cette glycoprotéine :
- la démannosylation de l'Apa obtenue par traitement de l'Apa native par l'α-mannosidase ou par l'acide trifluorométhane sulfonique (TFMS) ou bien par expression de l'Apa chez une bactérie incapable de glycosyler *(E. coli)* s'accompagne d'une perte d'antigénicité d'un facteur 100,
- l'Apa glycosylée, produite par *Mycobacterium smegmatis* qui a une composition globale en mannose légèrement différente de celle de l'Apa produite par M. *tuberculosis* a une activité antigénique diminuée d'environ un facteur 10.

Par ailleurs, il a été proposé d'utiliser des polypeptides comprenant une fraction immunogène d'un antigène soluble de *M. tuberculosis* dénommé DPEP, correspondant à l'Apa, comme vaccin ou réactif de diagnostic de la tuberculose (Demande Internationale PCT WO 98/16646).

Il a également été proposé d'utiliser une préparation d'antigènes comprenant l'Apa (MPT-32) ou des fragments de l'Apa comprenant un épitope B capable de se lier aux anticorps d'un individu atteint de tuberculose pour l'immunodétection rapide et précoce de la tuberculose (Demande Internationale WO 98/29132).

La glycosylation de l'Apa a été analysée à partir de glycopeptides [Apa 1-12, Apa 13-19, Apa 248-282 (correspondant à la séquence SEQ ID NO : 11 dans le listage des séquences annexé) et Apa 269-281] obtenus par digestion enzymatique de l'Apa par la subtilisine ou un mélange chymotrypsine/trypsine. Cette analyse a montré que la glycoprotéine Apa de *M. tuberculosis* contient 6 à 9 résidus mannoses liés par une liaison glycosidique de type α-(1,2) à 4 résidus thréonine (T₁₀, T₁₈, T₂₇ et T₂₇₇) de la façon suivante: un di-mannose (T₁₀ et T₁₈), un mannose (T₂₇), un mannose, un di-mannose ou un tri-mannose (T₂₇₇), (Dobos et al., J. Bacteriol., 1996, 178, 2498-2506*).* En outre, de tels glycopeptides O-glycosylés comprenant un dimannose (α-D-Man(1→ 2)-D-Man) peuvent être synthétisés à partir d'acides aminés O-glycosylés, selon la méthode décrite dans Franzyk et al., J. Chem. Soc. Perkin Trans., 1995, 22, 2883-2898.

Il faut noter que la structure osidique de l'Apa qui contient des mono-, di- ou tri-mannoses liés à des résidus thréonineressemble à celle des mannoprotéines de levure, en particulier de *Candida albicans,* et est différente de celle des protéines de F. *meningosepticum* qui possèdent des chaînes oligomannosiques plus longues. Cette structure osidique de l'Apa de *M. tuberculosis* (glycoprotéine O-glycosylée) est également différente de celle des antigènes O-glycosylés de cellules de mammifère (mucine ; Demande Internationale PCT WO 99/34824) qui comprennent un résidu N-acetylgalactosamine (GalNAc) suivi d'autres sucres (galactose) liés à des résidus de thréonine, et de celle des antigènes N-glycosylés des virus (complexe gcI du cytomegalovirus humain ; Brevet américain US 5,124,440).

La perte de l'antigénicité de l'Apa, observée après démannosylation, pourrait être due à une diminution de la phagocytose et, de l'apprêtement de cet antigène ou bien de la reconnaissance de celui-ci par les lymphocytes T CD4+. En effet, le récepteur au mannose des macrophages et des cellules dendritiques qui se lie spécifiquement aux hexoses, notamment des mannoprotéines de *C. albicans,* et des mannolipides comme le lipoarabinomannane des mycobactéries, joue un rôle dans la phagocytose et l'apprêtement des antigènes qui sont présentés à la surface de ces cellules sous forme d'un complexe peptide-molécule de classe II du CMH. (Stahl et al., Current Opinion in Immunology, 1998, 10, 50-55*).* Il a aussi été montré qu'un peptide mannosylé (au niveau de résidus lysine en position N-terminale) était phagocyté et apprêté par les cellules dendritiques, de façon beaucoup plus efficace qu'un peptide non-glycosylé de même séquence (Tan et al., Eur. J. Immunol., 1997, 27, 2426-2435*).*

Dans le modèle du lysozyme de la poule, il a été montré que des peptides analogues glycosylés d'un peptide constituant un épitope T de cet antigène étaient capables d'induire des lymphocytes T CD4+ reconnaissant spécifiquement cet épitope glycosylé (Deck et al., J. Immunol., 1995, 155, 1074-1078). Toutefois, dans la mesure où de tels épitopes T glycosylés reconnus spécifiquement par des lymphocytes T CD4+ n'ont pas été identifiés dans des antigènes natifs issus de microorganismes pathogènes (bactérie-champignon), l'importance de la glycosylation dans la reconnaissance des antigènes de *M. tuberculosis* reste à démontrer.

En outre, et ce malgré les données relatives à l'Apa de *M. tuberculosis* et les connaissances générales sur la glycosylation des antigènes, il n'a pas été possible jusqu'à présent de préparer des antigènes issus des protéines O-glycosylées de ce microorganisme pathogène, aptes à être effectivement utilisables dans une composition immunogène ou vaccinale et/ou dans un test de diagnostic.

En effet :
- les protéines actives qui ne représentent qu'un faible pourcentage des protéines produites par ce microorganisme sont purifiées avec des rendements très faibles, par des procédés qui sont dangereux, du fait de la manipulation de grandes quantités de ces agents pathogènes,
- les protéines, produites dans des systèmes d'expression hétérologues (cellules eucaryotes ou bactéries incapables de glycosyler), ont une activité antigénique faible,
- les protéines produites dans des systèmes d'expression homologue comme *M. smegmatis* ont une activité antigénique acceptable mais elles sont produites en quantités insuffisantes par des procédés complexes.

En conséquence, les Inventeurs se sont donnés pour but de préparer des antigènes immunodominants, aptes à induire une réponse immunitaire protectrice humorale et/ou cellulaire, qui, d'une part, administrés seuls ou en combinaison avec d'autres antigènes, pourraient constituer un vaccin utilisable chez tous les individus incluant les sujets immunodéprimés (disparition du risque lié à l'utilisation d'un vaccin vivant), d'autre part pourraient être utilisés à des fins diagnostiques.

Ils ont trouvé que certains glycopeptides issus de *M. tuberculosis,* un microorganisme pathogène synthétisant des glycoprotéines présentent une activité antigénique au moins égale sinon supérieure à celle de la protéine native déglycosylée ou de la protéine recombinante produite dans *E*. *coli.*

C'est également un but de l'invention de développer des moyens simples à mettre en oeuvre pour la production de ces glycopeptides en grandes quantités.

La présente invention a pour objet des glycopeptides immunogènes de 15 à 39 acides aminés issus de *M. tuberculosis,* lesquels glycopeptides comprennent un épitope T glycosylé de 14 à 25 acides aminés, parmi lesquels au moins un acide aminé neutre est lié à un di- ou à un trimannose (liaison glycosidique) et au moins 15 % d'entre lesdits acides aminés sont des prolines, l'une des proline étant située en position -1 à -4, relativement à la position dudit acide aminé neutre, lesquels glycopeptides sont :
- présentés par une molécule de classe II du CMH,
- reconnus spécifiquement par des lymphocytes T CD4+ induits par immunisation avec la glycoprotéine native dont ils sont issus, mais ne sont pas reconnus par les lymphocytes T CD4+ induits par immunisation avec un peptide non glycosylé de même séquence et
- capables d'induire une prolifération desdits lymphocytes T CD4+ par lesquels ils sont reconnus et la sécrétion de cytokines par lesdits lymphocytes, à l'exclusion du glycopeptide de séquence SEQ ID NO:11, issu de l'Apa qui est décrit par Dobos et al. (J. Bacteriol." 1996, 178, 2498-2506).

La présente invention a également pour objet des glycopeptides constitués par un épitope T glycosylé de 14 à 25 acides aminés tel que défini ci-dessus.

Ces glycopeptides constitués essentiellement par un épitope T glycosylé sont reconnus par des lymphocytes T CD4+ par l'intermédiaire de cet épitope T glycosylé. En effet, après immunisation avec des bacilles vivants du groupe de la tuberculose, les lymphocytes T spécifiques de ces glycopeptides sont beaucoup plus nombreux que les lymphocytes T spécifiques des peptides non glycosylés de même séquence.

De manière avantageuse, lesdits glycopeptides ont une activité antigénique au moins 10 fois supérieure, de préférence au moins 30 fois supérieure, à celle d'un peptide témoin de même séquence.

Lesdits glycopeptides présentent les avantages suivants :
- induction d'une réponse immunitaire de type cellulaire protectrice et éventuellement d'une réponse humorale, et utilisation possible comme antigènes chez les sujets immunodéprimés.
- activité antigénique au moins égale sinon supérieure aux antigènes classiques (culture dudit microorganisme vivant atténué, mélanges d'antigènes préparés à partir desdites cultures ou peptides non glycosylés) car ils sont reconnus par un nombre plus important de lymphocytes T spécifiques de *M. tuberculosis,*
- spécificité très étroite, qui permet à la fois d'éliminer les problèmes de réactivités croisées avec d'autres microorganismes, notamment avec d'autres mycobactéries atypiques, et d'augmenter l'efficacité de la vaccination et du diagnostic de *M*. *tuberculosis ;* en effet, ils sont plus spécifiques étant donné que leurs résidus oligosaccharidiques, présents exclusivement dans ledit microorganisme pathogène, participent de façon cruciale à la définition de l'épitope T reconnu par les lymphocytes T CD4⁺; ils constituent ainsi des antigènes spécifiques pour la vaccination et le diagnostic de l'infections par *M. tuberculosis,* un organisme pathogène apte à O-glycosyler certaines de ses protéines.
- utilisation chez les sujets immunodéprimés car ils sont totalement apathogènes,
- production possible en grandes quantités,
- meilleure standardisation des doses actives et de l'efficacité du vaccin,
- facilités de stockage et d'utilisation,

Selon un mode de réalisation avantageux desdits glycopeptides, ledit acide aminé neutre est sélectionné dans le groupe constitué par la sérine et la thréonine.

Selon une disposition avantageuse de ce mode de réalisation desdits glycopeptides, ils contiennent de 1 à 7 résidus thréonine, liés à un di- ou à un trimannose.

Selon encore un autre mode de réalisation avantageux desdits glycopeptides, le di-ou trimannose comprend une liaison α-(1,2).

Conformément à l'invention, lesdits glycopeptides sont, de préférence, issus :
- de la protéine Apa de *M. tuberculosis* (Genbank numéro X80268) ou
- de la protéine Rv1796 codée par le gène *Rv 1796*, en référence à l'annotation de la séquence du génome de *M. tuberculosis* de souche H37Rv (Banque Sanger).

De préférence, ledit glycopeptide est sélectionné dans le groupe constitué par :
- un glycopeptide de 39 acides aminés dont la séquence (SEQ ID NO:1) est celle qui s'étend des positions 1 à 39 de la séquence de la protéine Apa et dans laquelle au moins un des résidus thréonine en position 10, 18 et 27 de la SEQ ID NO: 1 est lié à un di- ou trimannose par une liaison glycosidique,
- un glycopeptide de 26 acides aminés dont la séquence (SEQ ID NO:2) est celle qui s'étend des positions 261 à 286 de la séquence de la protéine Apa (séquence C-terminale) et dans laquelle le résidu thréonine en position 17 de la SEQ ID NO:2 est lié à un di- ou trimannose par une liaison glycosidique, et
- un glycopeptide de 35 acides aminés dont la séquence (SEQ ID NO:3) est celle qui s'étend des positions 169 à 203 de la séquence de la protéine Rv 1796 et dans laquelle au moins un des résidus thréonine en position 4, 5, 7, 13, 15, 23 et 25 de la SEQ ID NO:3 est lié à un di- ou trimannose par une liaison glycosidique.

L'invention a également pour objet un procédé de synthèse d'un glycopeptide tel que défini précédemment, caractérisé en ce qu'il comprend les étapes suivantes :
- préparation, en solution, d'acides aminés neutres glycosylés, liés à un di- ou à un trimannose par une liaison glycosidique,
- synthèse, sur support solide, du glycopeptide à l'aide des acides aminés nécessaires à l'obtention de la séquence peptidique dudit glycopeptide et des acides aminés neutres glycosylés, obtenus précédemment, et
- coupure du glycopeptide du support solide.

Selon un mode de mise en oeuvre avantageux dudit procédé, ledit acide aminé neutre est sélectionné dans le groupe constitué par la sérine et la thréonine.

Selon une disposition avantageuse de ce mode de mise en oeuvre, lorsque lesdits glycopeptides présentent les séquences suivantes (**T** représente une thréonine O-glycosylée, fonctionnalisée par 2 ou 3 résidus mannose et Ac représente une fonction acétate) :
SEQ ID NO:1 : H₂N-DPEPAPPVP**T**TAASPPS**T**AAAPPAPA**T**PVAPPPPAAANT-CONH₂
SEQ ID NO:2 : AcNH-PAPAPAPAGEVAPTPT**T**PTPQRTLPA-COOH
SEQ ID NO:3 : AcNH-TIP**TT**ETPPPPQ**T**V**T**LSPVPPQ**T**V**T**VIPAPPPEEG-CONH₂,
ledit procédé comprend les étapes suivantes :
i) préparation, en solution, de thréonines O-glycosylées fonctionnalisées par 2 ou 3 résidus mannose,
ii) synthèse, sur support solide, des peptides correspondant aux séquences SEQ ID NO:1, SEQ ID NO:2 et SEQ ID NO:3 sus-mentionnées, à l'aide des acides aminés nécessaires à l'obtention de ces séquences et des thréonines O-glycosylées obtenues lors de l'étape i),
iii) coupure des peptides du support solide, et
iv) introduction, par synthèse chimique, d'une fonction amide au niveau de l'extrémité C-terminale des peptides SEQ ID NO:1 et SEQ ID NO:3, et d'une fonction acétate au niveau de l'extrémité N-terminale des peptides SEQ ID NO:2 et SEQ ID NO:3.

La synthèse des peptides SEQ ID NO:1, SEQ ID NO:2 et SEQ ID NO:3 correspond donc à une synthèse peptidique classique en phase solide, au cours de laquelle sont introduits des acides aminés glycosylés. Comme connu dans le domaine de la synthèse peptidique en phase solide, les acides aminés utilisés sont convenablement protégés et, si nécessaire, activés avant d'être incorporés l'un après l'autre dans la séquence peptidique. De même, les hydroxyles présents au niveau des résidus mannose portés par les thréonines doivent être convenablement protégés au cours de la synthèse peptidique.

Une fois la synthèse peptidique effectuée, les peptides sont séparés du support solide et déprotégés. Ils peuvent être purifiés par Chromatographie Liquide Haute Performance (HPLC) en phase inverse.

Selon une disposition avantageuse de ce mode de mise en oeuvre, les résidus mannose portés par les thréonines O-glycosylées préparées lors de l'étape i) sont reliés entre eux par des liaisons α-(1,2).

Selon une modalité avantageuse de cette disposition, les thréonines fonctionnalisées par des résidus mannose sont préparées comme suit :
a₂) préparation de dérivés du mannose de formules (I) et (II) : 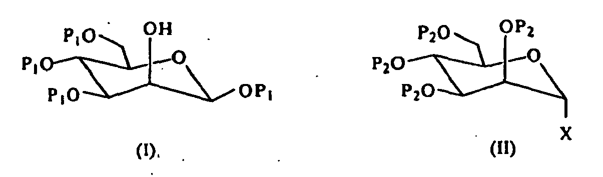 dans lesquelles P₁ et P₂, qui peuvent être identiques ou différentes, représentent des groupes protecteurs d'une fonction hydroxyle, et X représente une fonction activée, telle qu'un atome de brome,
b₂) réaction du dérivé de formule (I) avec le dérivé de formule (II), puis activation du composé obtenu, conduisant à l'obtention d'un dérivé activé comportant deux résidus mannose et répondant à la formule (III) : 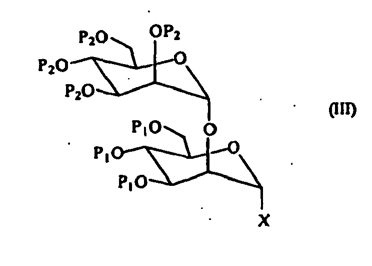 dans laquelle P₁, P₂ et X sont tels que définis en rapport avec les formules (I) et (II),
c₂) éventuellement, réaction du composé de formule (III) avec un dérivé du mannose de formule (I) telle que définie en a₂), puis activation du composé obtenu, conduisant à l'obtention d'un dérivé activé comportant trois résidus mannose et répondant à la formule (IV) : 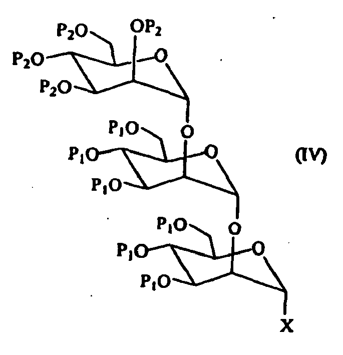 dans laquelle P₁, P₂ et X sont tels que définis en rapport avec les formules (I) et (II), et
d₂) condensation du composé de formule (III) ou du composé de formule (IV) avec une thréonine convenablement protégée de formule (V) : 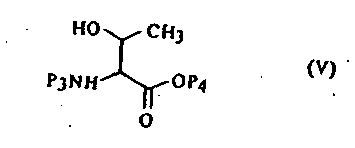 dans laquelle P₃ représente un groupe protecteur d'une fonction amine primaire et P₄ représente un groupe protecteur d'une fonction carboxyle,
   conduisant respectivement à l'obtention d'une thréonine glycosylée de formule (VI) ou (VII) : 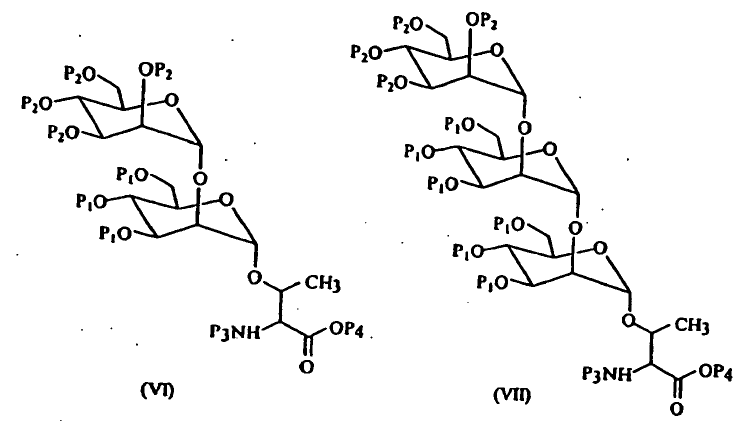 dans lesquelles P₁, P₂, P₃ et P₄ sont tels que définis précédemment.

Les groupes protecteurs P₁, P₂, P₃ et P₄ peuvent être choisis parmi ceux décrits dans l'ouvrage Protective Groups in Organic Synthesis, T. W. GREENE et P.G.M. WUTS, Seconde Edition, 1991, J. WILEY and Sons. A titre d'exemples et de façon non-limitative, P₁ et P₂ peuvent représenter des groupes acétyle ou benzoyle, P₃ peut représenter un groupe Fmoc (fluorène-9-yl-méthoxycarbonyle) et P₄ peut représenter un groupe pentafluorophényle.

La présente invention a également pour objet un procédé de sélection et de criblage de glycopeptides immunogènes à partir de la séquence peptidique de glycoprotéines de *M. tuberculosis,* qui peut avantageusement être mis en oeuvre de façon concomitante au procédé de synthèse des glycopeptides conformes à l'invention, tel que défini ci-dessus, lequel procédé est caractérisé en ce qu'il comprend au moins les étapes suivantes :
a₃) recherche et sélection dans la séquence peptidique des dites glycoprotéines d'au moins une séquence de 14 à 25 acides aminés, contenant au moins un acide aminé neutre lié à un di- ou un trimannose et au moins 15% de proline, l'une des prolines étant située en position -1 à -4, relativement à la position dudit acide aminé neutre,
b₃) préparation du/des glycopeptide(s) sélectionné(s) à l'étape a₃), conformément au procédé de synthèse défini ci-dessus, et
c₃) sélection des glycopeptides dont l'activité antigénique est au moins 10 fois supérieure, de préférence au moins 30 fois supérieure à celle d'un peptide témoin de même séquence.

Selon un mode de mise en oeuvre avantageux dudit procédé de criblage, préalablement à l'étape a₃), il comprend une étape de présélection d'au moins une glycoprotéine antigénique.

Selon encore un mode de mise en oeuvre avantageux dudit procédé de criblage, à l'étape c₃), l'activité antigénique dudit glycopeptide est évaluée par mesure de l'activation des lymphocytes T CD4+ d'animaux immunisés par *Mycobacterium bovis* BCG ou par une fraction antigénique de *M. tuberculosis.*

L'activation des lymphocytes T peut-être mise en évidence par des techniques classiques d'immunologie telle que celles décrites dans Current protocols in Immunology (John E. Coligan, 2000, Wiley and son Inc, Library of Congress, USA*).* A titre d'exemple on peut citer les tests de prolifération lymphocytaire, les dosages des cytokines (protéine ou ARNm) synthétisées par des lymphocytes T CD4+ activés (immunoessai (ELISA) ou réaction de polymérisation en chaîne de type RT-PCR) ou les tests d'hypersensibilité de type retardé.

La présente invention englobe également les glycopeptides susceptibles d'être obtenus par le procédé de sélection et de criblage tel que défini ci-dessus.

La présente invention a également pour objet l'utilisation d'au moins un glycopeptide conforme à l'invention ou d'un glycopeptide de séquence SEQ ID NO:11, pour la préparation d'une composition immunogène ou vaccinale ou d'un réactif de diagnostic.

La présente invention a également pour objet une composition immunogène apte à induire une immunité humorale et/ou cellulaire, caractérisée en ce qu'elle comprend au moins un glycopeptide tel que défini ci-dessus, associé à au moins un véhicule pharmaceutiquement acceptable.

Du fait de la coopération entre les lymphocytes T CD4+ et les lymphocytes T CD8+ ou les lymphocytes B dans la mise en place d'une réponse immunitaire humorale ou cellulaire, les glycopeptides de l'invention peuvent avantageusement être utilisés comme protéine de transport (« *carrier»*) de tout autre antigène vaccinal pour augmenter l'efficacité de l'immunisation contre ledit antigène. Cette association antigène-carrier permet avantageusement de faciliter la sélection et l'amplification des lymphocytes B et T spécifiques de l'antigène vaccinal.

La présente invention a également pour objet une composition vaccinale apte à induire une immunité humorale et/ou cellulaire, caractérisée en ce qu'elle comprend au moins un glycopeptide tel que défini ci-dessus, associé à au moins un véhicule pharmaceutiquement acceptable, et éventuellement à au moins un adjuvant.

Selon un mode de réalisation avantageux desdites compositions immunogène ou vaccinale, ledit glycopeptide est associé à une protéine ou un fragment de protéine comprenant au moins un épitope B, un épitope T de type CD4+ ou un épitope T de type CD8+.

Au sens de la présente invention, on entend par épitope B, épitope T de type CD4⁺ et épitope T de type CD8⁺ relativement à la séquence d'une protéine; le fragment de cette séquence qui est capable de se lier respectivement à un anticorps, à un récepteur T de lymphocytes CD4+ et à un récepteur T de lymphocytes CD8+.

Au sens de la présente invention on entend par association du glycopeptide à une protéine aussi bien le mélange que le couplage par tout moyen physique ou chimique, par exemple l'expression d'une fusion entre la séquence du glycopeptide et celle de la protéine ou du fragment de protéine.

Les adjuvants utilisés sont des adjuvants classiquement utilisés, avantageusement ils sont choisis dans le groupe constitué par l'hydroxyde d'alumine et le squalène.

Ledit glycopeptide peut éventuellement être associé à tout autre moyen, en lui-même connu de l'homme du métier, permettant d'augmenter l'immunogénicité d'un peptide. A titre d'exemple on peut citer le couplage à un peptide porteur qui permet l'obtention d'un peptide multimérisé ramifié, tel que celui décrit par Wilkinson et al., 1999, Eur. J. Immunol., 29,2788-2796.

La présente invention a également pour objet des anticorps, caractérisés en ce qu'ils sont dirigés contre l'un ou plusieurs des glycopeptides selon la présente invention.

Selon un mode de réalisation avantageux desdits anticorps, ils sont sélectionnés parmi les anticorps monoclonaux et les anticorps polyclonaux.

La présente invention a en outre pour objet un réactif de diagnostic, caractérisé en ce qu'il est sélectionné dans le groupe constitué par les glycopeptides et les anticorps selon l'invention.

Les glycopeptides selon l'invention qui détectent de façon très spécifique l'immunité cellulaire et/ou humorale induite par l'infection par *M. tuberculosis,* peuvent avantageusement être utilisés pour le diagnostic de la tuberculose par toute technique permettant la détection de l'immunité humorale et/ou cellulaire, cette technique étant en elle-même connue de l'homme du métier. A titre d'exemple, on peut citer les tests de prolifération de lymphocytes T et les dosages immunoenzymatiques de cytokines spécifiques des lymphocytes T CD4+, notamment l'INF-γ.

La présente invention a également pour objet un procédé de détection de la tuberculose, caractérisé en qu'il comprend la détection, *in vitro,* de lymphocytes T CD4+ reconnaissant un glycopeptide tel que défini ci-dessus, par toute technique appropriée.

La présente invention a également pour objet un procédé de détection de la tuberculose, caractérisé en qu'il comprend la mise en contact d'un échantillon biologique d'un patient susceptible d'être infecté par *M. tuberculosis,* avec un réactif de diagnostic tel que défini ci-dessus (anticorps ou glycopeptides, selon le cas) et la détection de la formation d'un complexe anticorps et microorganisme présent dans l'échantillon biologique ou glycopeptide(s) et anticorps présents dans l'échantillon.

Outre les dispositions qui précèdent, l'invention comprend encore d'autres dispositions, qui ressortiront de la description qui va suivre, laquelle se réfère à des exemples de mise en oeuvre de la présente invention ainsi qu'aux dessins annexés dans lesquels :

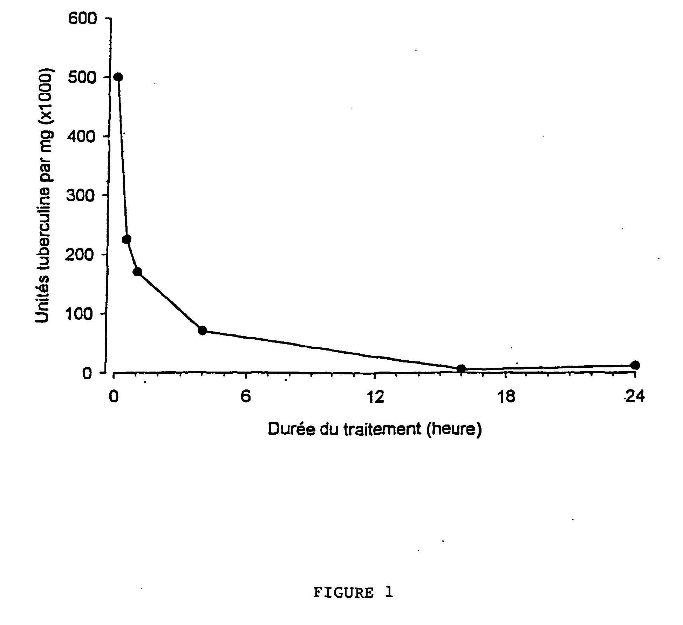

- la figure 1 illustre la mesure par un test d'hypersensibilité de type retardé, de l'activité antigénique de l'Apa native purifiée *de M. tuberculosis,* en fonction de la cinétique de digestion de la protéine Apa par l'α-mannosidase. Les résultats sont exprimés en unités de tuberculine par mg de protéine en fonction du temps en heures,

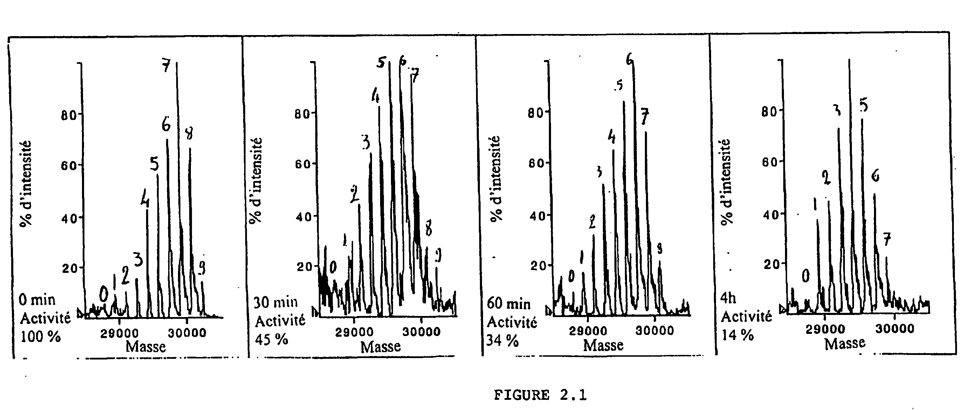

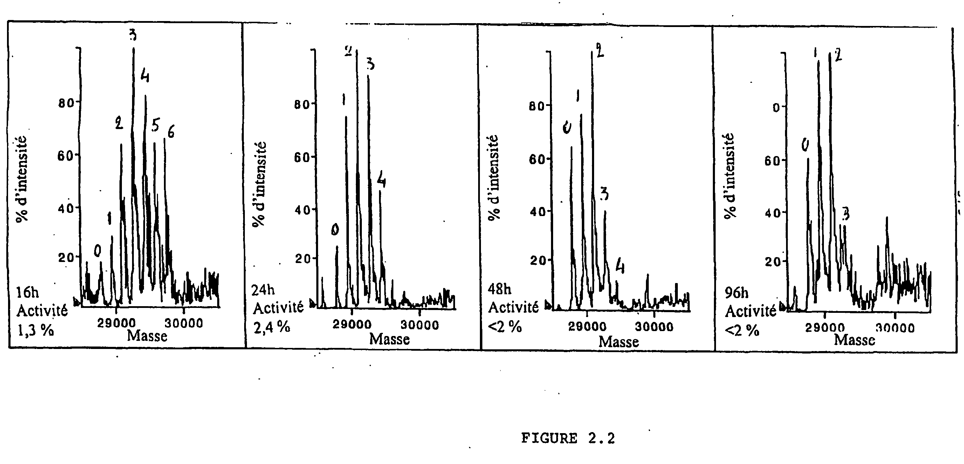

- la figure 2 illustre l'analyse par spectrométrie de masse de la composition en mannose des molécules d'Apa, en fonction de la cinétique de digestion de la protéine Apa par l'α-mannosidase. Le nombre de résidus mannose correspondant à chaque pic de la protéine Apa est indiqué et l'activité antigénique globale du produit de la digestion de l'Apa est indiquée aux différents temps étudiés,

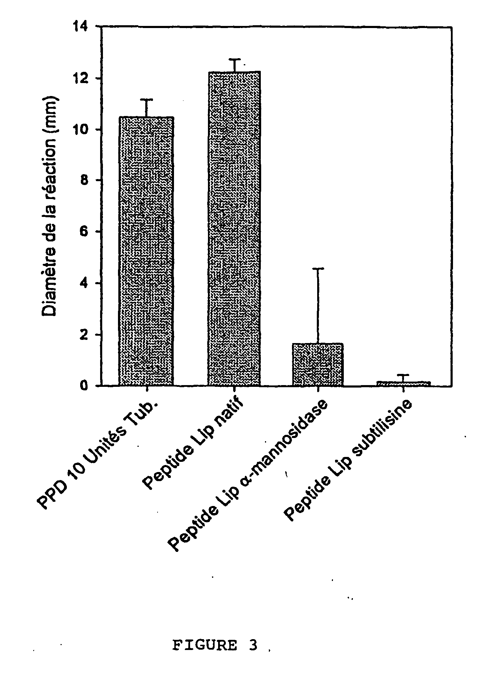

- la figure 3 illustre la mesure par un test d'hypersensibilité de type retardé, de l'activité antigénique d'un glycopeptide, dénommé Lip, issu de la protéine Rv 1796 (SEQ ID NO:3). Les protéines standard purifiées de *M. tuberculosis* (PPD) sont utilisées comme témoin positif à la dose de 0,25 µg dans 0,1 ml. Le peptide Lip est utilisé à la dose de 0,02 µg dans 0,1 ml. Les peptides Lip traités par l'α-mannosidase ou la subtilisine sont négatifs aux mêmes doses. Les résultats sont exprimés par le diamètre de la réaction d'érythème,

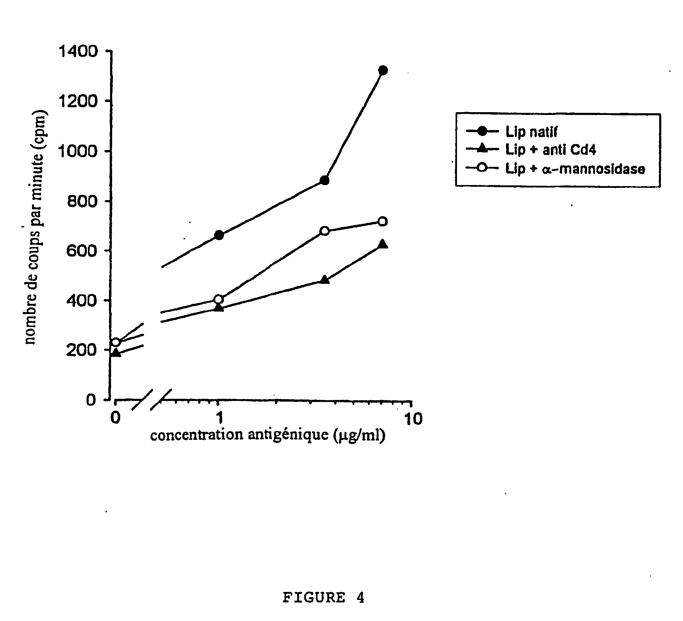

- la figure 4 illustre l'activité antigénique du peptide Lip par un test *in vitro* de prolifération lymphocytaire. La reconnaissance par les lymphocytes T du peptide Lip glycosylé (Lip natif) est comparée à celle du peptide déglycosylé (Lip + α-mannosidase) ou du peptide Lip associé à un anticorps anti-récepteur CD4+ des lymphocytes T (Lip + anti Cd4),

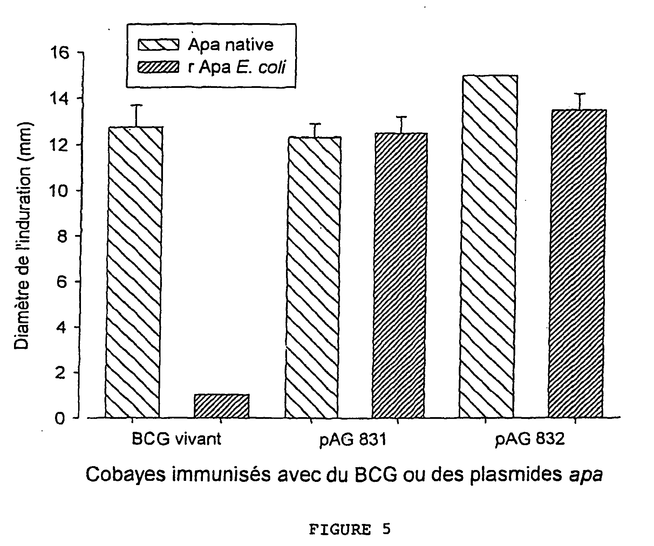

- la figure 5 illustre la mesure par un test d'hypersensibilité de type retardé de l'activité antigénique de l'Apa native purifiée de *M*. *tuberculosis* (Apa native)
ou de l'Apa recombinante déglycosylée produite dans *E*. *coli* (rApa *E*. *coli*)*,* en fonction de l'immunisation des cobayes. Ceux-ci ont été immunisés au préalable par le BCG vivant injecté en intradermique ou par les plasmides pAG831 ou pAG832, contenant la séquence codante de l'Apa, placée sous le contrôle du promoteur précoce du cytomégalovirus. L'immunisation des cobayes par les plasmides conduit à une sensibilisation pouvant être révélée par une réaction d'hypersensibilité de type retardé. Les deux types d'antigènes sont équivalents pour engendrer cette réaction, alors qu'après une immunisation par le BCG, seule l'Apa native glycosylée est antigénique.
- la figure 6 représente la préparation d'unités comportant deux ou trois résidus mannose reliés par des liaisons α-(1,2), et
- la figure 7 (7a et 7b) représente la préparation de thréonines fonctionnalisées par deux ou trois unités de mannose.

### EXEMPLE 1: Importance du nombre de résidus oligosaccharidiques dans l'antigénicité de la protéine Apa.

### 1. Matériels et méthodes

### a) Déglycosylation ménagée de l'Apa par digestion à l'α-mannosidase

450 µg de protéine Apa purifiée à partir du surnageant de culture de *M. tuberculosis,* selon le protocole décrit par Horn et al., précité sont dilués dans un volume de 450 µl de tampon A (100 mM CH₃COO⁻Na⁺, 2 mM ZnCl₂).

Au temps initial, 75 µl de la solution de protéine Apa sont prélevés, dilués dans 25 µl de tampon A et congelés comme témoin. 125 µl d'α-mannosidase à 1 mg/ml (3 UI/ml, Oxford Glycosciences) sont ensuite ajoutés au 375 µl de la solution d'Apa et le volume réactionnel de 500 µl est incubé à 37°C. Après 30 min, 1 h, 4 h, 16 h et 24 h, 100 µl de la réaction sont prélevés et congelés à -20°C.

### b) Purification des produits de digestion

Les échantillons de 100 µl sont chauffés 2 min à 90°C puis ils sont refroidis brusquement, séchés par le vide et remis en suspension dans 300 µl d'acide tri-fluoro-acétique à 0,1% dans l'eau (solution B).

Les produits de digestion de l'Apa sont séparés de l'α-mannosidase sur une colonne de chromatographie en phase réverse (Ressource RPC, Pharmacia), par un gradient de 0 à 90 % d'acétonitrile dans la solution B, en 90 min. L'Apa est éluée de la colonne au temps t = 68 min, correspondant à 51,5% ± 0,5% d'acétonitrile. Les fractions correspondant à l'Apa sont collectées, lyophilisées, remises en suspension dans une solution de butanol à 5% dans l'eau (solution C), puis séchées sous vide. Les échantillons purifiés sont alors remis en suspension dans 100 µl de solution C.

### c) Analyse biochimique des produits de digestion de l'Apa

La composition oligosaccharidique de chaque échantillon est analysée par spectrométrie de masse, dans les conditions décrites dans Horn et al., précité.

Une mesure de l'absorption à 210 nm est effectuée afin d'évaluer la quantité relative de protéine présente dans chaque échantillon.

Ensuite, les échantillons sont séchés et leur concentration est ajustée à 1 mg/ml dans un tampon de titrage (tampon D : PBS, 0,9% NaCl, 0,05% Tween 80).

### d) Titrage biologique de l'activité antigénique des produits de digestion ménagée de l'Apa par l'α-mannosidase dans un test d'hypersensibilité de type retardé

L'activité antigénique est mesurée par un test d'hypersensibilité de type retardé sur des cobayes immunisés 3 mois auparavant par une injection intradermique de 2 mg de BCG vivant en 2 points d'injection.

Chaque échantillon est dilué à une concentration de 2 µg/ml dans le tampon D et 100 µl de cette dilution (0,2 µg) sont injectés par voie intradermique à des lots de 2 cobayes préalablement immunisés.

Les différents lots d'animaux sont les suivants :
- lot 1 : témoin négatif ayant reçu 100 µl de tampon D
- lot 2 : Apa t=0
- lot 3 : Apa t=30 min
- lot 4 : Apa t=1 h
- lot 5 : Apa t= 4h
- lot 6 Apa t=16 h
- lot 7 Apa t= 24h
- lot 8 : témoin positif (0,25 µg de protéines standard purifiées de *Mycobacterium tuberculosis* (PPD) correspondant à 10 unités de tuberculine (UT).

24 h après l'injection, la moyenne du diamètre de la réaction d'érythème est mesurée pour les différents lots d'animaux et le titre en tuberculine des échantillons est déterminé par rapport au standard PPD.

### 2. Résultats

Les résultats sont illustrés par les figures 1 et 2.

L'analyse de l'activité antigénique de l'Apa en fonction de la cinétique de digestion par l'α-mannosidase (figure 1) montre que l'activité antigénique de l'Apa est progressivement perdue au cours de la digestion par l'α-mannosidase : 66% en 1 h, 86% en 4 h et 97 à 99% pour les digestions plus longues.

L'analyse de la composition en mannose des produits obtenus aux différents temps de digestion (figure 2) montre que :
- les molécules d'Apa natives possèdent 6 à 8 résidus mannose, et
- les molécules d'Apa sur lesquelles persistent 3 à 6 résidus mannose perdent 86 % de leur activité antigénique.

Il a été montré que la composition en oligomannose de l'Apa est la suivante : un di-mannose (T₁₀ et T₁₈), un mannose (T₂₇), un mannose, un di-mannose ou un tri-mannose (T₂₇₇), Dobos et al., précité. En outre, l'α-mannosidase est une exomannosidase.

Par conséquent, les résultats indiquent que :
- la perte de 1 ou 2 des mannoses terminaux des 4 chaînes oligomannose de l'Apa entraîne une perte drastique de l'activité antigénique, et
- l'antigénicité de l'Apa est liée à la présence d'un di- ou d'un tri- mannose sur un ou plusieurs des résidus thréonine glycosylés.

### EXEMPLE 2 : Mise en évidence du glycopeptide Lip de M. tuberculosis.

### 1. Matériels et méthodes

### a) Purification du glycopeptide

### a1) Préparation du matériel brut

Des bactéries de la souche de *Mycobacterium tuberculosis* (H37Rv) sont cultivées durant 20 jours sur un milieu synthétique de Sauton (Milieux de culture, H. Cassagne, 1961, Ed. Institut Pasteur, Tome 2, page 242). Les molécules sécrétées dans le milieu sont concentrées sur une membrane d'ultrafiltration (PM10, AMICON) de façon à ne retenir que les molécules de masse moléculaire supérieure à 10 000 Da, puis elles sont lyophilisées. Il est obtenu environ 10 g de lyophilisat pour 60 litres de milieu de culture.

### a2) Filtration moléculaire (étape 1)

Une colonne préparative est chargée avec la matrice Sup75 prepgrade Pharmacia. Cette colonne de 50x750 mm est équilibrée avec un tampon phosphate (50 mM PO₄Na₂/K, pH 7,1 ; 100 mM NaCl ; 4 % butanol) à un débit de 1 ml/min. Le matériel brut précédent est repris dans le tampon d'équilibre à une concentration finale de 10 g pour 100 ml, clarifié par centrifugation à 43 000 g, durant 4h puis par filtration sur filtre 0,22 µm. Des injections de 13 ml sont effectuées et les différentes fractions détectées par leur absorbance à 280 nm sont concentrées sur membrane PM10 puis lyophilisées.

La fraction éluée entre 700 et 800 ml est très antigénique : on observe une hypersensibilité de type retardé chez des cobayes immunisés par du BCG vivant ; cette fraction est par contre peu active chez des cobayes immunisés par du BCG inactivé par la chaleur.

### a3) Echange d'ions (étape 2)

Une colonne préparative Source 15Q Pharmacia (15 µm) de 24x250 mm est équilibrée avec un tampon 20 mM Tris/HCl, pH 8, 4 % butanol, à un débit de 5 ml/min avec une pression maximale de 8 bars. Un gradient linéaire de NaCl de 0 à 150 mM dans le même tampon est appliqué après injection de 500 mg de la fraction précédente dissoute dans 10 ml du tampon initial. Les fractions éluées sont détectées par absorption à 280 nm, concentrées sur membrane PM10 puis lyophilisées.

La fraction éluée, entre 40 et 75 mM de NaCl est très antigénique : on observe une hypersensibilité de type retardé chez des cobayes immunisés par du BCG vivant ; cette fraction est par contre peu active chez des cobayes immunisés par du BCG inactivé par la chaleur.

### a4) Phase inverse sur colonne C8 (étape 3)

Une colonne RPC (Reversed Phase Column) Resource 15RPC de 4,6x100 mm Pharmacia est équilibrée par un tampon 20 mM CH3COO⁻NH4⁺, pH 6,5, à un débit de 1 ml/min. Un gradient non linéaire d'acétonitrile, compris entre 0 et 90 %, est appliqué après l'injection sur la colonne de 10 mg dans 2 ml de tampon de la fraction précédente. Les fractions éluées sont détectées à 280 nm puis concentrées sous vide à 40°C avant d'être lyophilisées.

La fraction éluée entre les concentrations 18 et 22 % d'acétonitrile est très antigénique pour révéler une hypersensibilité de type retardé chez des cobayes immunisés par du BCG vivant et peu active chez des cobayes immunisés par du BCG inactivé par la chaleur.

### a5) Phase inverse sur colonne C18 (étape 4)

Une colonne microbore phase inverse C18 (Browlec lab. 1x250 mm) est équilibrée par un tampon 20 mM CH3COO⁻NH4⁺, pH 6,5, à un débit de 1 ml/min. Un gradient non linéaire d'acétonitrile de 0 à 90 % est appliqué après injection sur la colonne de la fraction précédente.

Une fraction détectée seulement à 220 nm est éluée par une concentration d'environ 11 % d'acétonitrile. Cette fraction (3 mg) est très active pour révéler les réactions d'hypersensibilité de type retardé chez des cobayes immunisés par des bactéries vivantes et peu active chez des cobayes immunisés par du BCG inactivé par la chaleur.

### b) Analyse biochimique du glycopeptide purifié

La fraction obtenue à l'étape finale de purification a été séquencée par une technique d'Edman modifiée (Applied Biosystems 473A), selon les instructions du fabricant.

La composition de chaque échantillon est analysée par spectrométrie de masse (spectromètre MALDI-TOF), dans les conditions décrites dans Horn et al., précité.

### c) Digestion du glycopeptide par l'α-mannosidase.

9 µg du glycopeptide purifié ci-dessus sont dissous dans 65 µg de tampon 100 mM CH₃COO⁻Na⁺, pH 5 puis 3 µl d'une solution à 1 mg/ml d'α-mannosidase (Oxford Glyco System), soit 90 mU d'α-mannosidase, sont ajoutés. La réaction est incubée 24h à 37°C de façon à obtenir une digestion totale, puis le produit obtenu est séché sous vide.

### d) Digestion du peptide par la subtilisine

690 ng du glycopeptide purifié ci-dessus sont dissous dans 5 µl de tampon 100 mM carbonate d'ammonium, pH 8, puis 1 µl d'une solution de subtilisine à 100 µg/ml soit environ 100 ng est ajouté. La réaction est incubée 24h à 37°C puis le produit de la réaction est séché sous vide et repris dans le tampon de titrage (tampon D).

### e) Titrage biologique de l'activité antigénique du glycopeptide par un test d'hypersensibilité de type retardé

0,02 µg du glycopeptide purifié ci-dessus, non-digéré ou digéré par l'α-mannosidase ou la subtilisine sont injectés à des lots de cobayes préalablement immunisés, selon le protocole décrit à l'exemple 1. Les résultats sont exprimés par la valeur du diamètre de la réaction d'érythème. Le témoin est constitué par 0,25 µg de PPD correspondant à 10 UT.

### f) Mesure de l'activité antigénique du glycopeptide par un test in vitro de prolifération lymphocytaire.

Les conditions du test sont celles décrites dans Horn et al., précité.

### 2. Résultats

### a) Purification et analyse biochimique du glycopeptide Lip

La mesure de masse effectuée sur le glycopeptide purifié indique la présence de molécules complexes, vraisemblablement glycosylées par des mannoses étant donné la présence de mesures différant d'une valeur de 162 unités de masse. Une masse de 6951 Da qui correspond à la masse du peptide traité à l'α-mannosidase est retenue comme la masse minimale de ces molécules.

La séquence N-terminale du glycopeptide purifié indique la présence d'une séquence majoritaire TIPTT... et d'une séquence minoritaire IPTTE....

Ces résultats sont compatibles avec un glycopeptide mannosylé, dénommé Lip dont la séquence (SEQ ID NO:3) est celle d'un fragment N-terminal d'un peptide issu de la protéine codée par le gène Rv1796 de *M*. *tuberculosis* qui s'étend des positions 169 à 239 de ladite protéine, en référence à l'annotation de la séquence du génome de *M. tuberculosis* de souche H37Rv de la banque de Sanger.

### b) Mesure de l'activité antigénique du glycopeptide Lip par un test d'hypersensibilité de type retardé

Le glycopeptide est très actif pour révéler les réactions d'hypersensibilité de type retardé chez des cobayes immunisés par des bactéries vivantes, en revanche il est peu actif chez des cobayes immunisés par du BCG inactivé par la chaleur.

L'activité antigénique du glycopeptide augmente au cours des étapes de purification :
- Etape 1 : La fraction obtenue a une activité de 180 000 (UT)/mg) chez des cobayes immunisés par du BCG vivant et de 10 000 UT/mg chez des cobayes immunisés par du BCG inactivé par la chaleur.
- Etape 2 : La fraction obtenue a une activité de 900 000 UT/mg chez des cobayes immunisés par du BCG vivant et de 30 000 UT/mg chez des cobayes immunisés par du BCG inactivé par la chaleur.
- Etape 3 : La fraction purifiée a une activité supérieure à 1 000 000 UT/mg chez des cobayes immunisés par du BCG vivant et inférieure à 30 000 UT/mg chez des cobayes immunisés par du BCG inactivé par la chaleur.

Les résultats illustrés à la figure 3 montrent que :
- l'action de l'α-mannosidase durant 24h à 37°C fait perdre plus de 95 % de l'activité antigénique : la fraction est passé d'une activité à 1 000 000 UT/mg à une activité inférieure à 30 000 UT/mg après déglycosylation,
- l'action de la subtilisine abolit l'activité antigénique, et
- à quantité équivalente de protéines, le glycopeptide Lip est au moins 10 fois plus actif que les protéines standard purifiées de *Mycobacterium tuberculosis* (PPD).

### c) Mesure de l'activité antigénique du glycopeptide Lip par un test in vitro de prolifération lymphocytaire.

Les résultats illustrés à la figure 4 montrent que la prolifération des lymphocytes T est dépendante de la concentration de peptide. Cette prolifération est marginale lorsque les lymphocytes T sont traités par un anticorps dirigé contre les molécules CD4 ou lorsque le glycopeptide est traité à l'α-mannosidase.

### EXEMPLE 3 : Mise en évidence du rôle des résidus oligosaccharidiques de l'Apa dans la définition d'épitopes T par immunisation avec de l'ADN nu codant pour la protéine Apa.

### 1. Matériel et Méthodes

### a) Construction d'un plasmide contenant la séquence codant pour la protéine Apa

Le plasmide pS65T (Clontech) contenant la séquence du promoteur précoce du cytomégalovirus est coupé par les enzymes de restriction NheI et BspEI, réparé par l'enzyme de Klenow puis ligaturé de façon à obtenir le plasmide pAG800.

Le plasmide pAG800 est coupé par l'enzyme ApaI et ligaturé avec l'oligonucléotide 12M48 (5' CAACGTTGGGCC 3'; SEQ ID NO:4) hybridé sur lui-même, pour donner le plasmide pAG802.

Un fragment de 875 paires de bases contenant la séquence codante de l'Apa dépourvue de la séquence signal est amplifié par réaction de polymérisation en chaîne (PCR) à partir du plasmide pLA34-2 (Laqueyrerie., 1995, Infect. Immun., 63, 4003-4010), en utilisant :
les oligonucléotides 22M42 (5'TCCCAAGCTTTTGGTAGCCG3', SEQ ID NO:5) et 33M44 (5'CTAGGATC CACCATGCCGGAGCCAGCGCCCCCG3', SEQ ID NO:6).

L'oligonucléotide 33M44 a été synthétisé de façon à contenir un site consensus d'initiation de la traduction de type Kozak (Nucl. Acids Res., 1987, 15, 8125-8148). Le fragment obtenu par PCR est coupé par BamHI et EcoRV et inséré dans le plasmide pAG802 coupé par BglII et SmaI, pour obtenir le plasmide pAG803. Au cours de ces opérations la séquence d'oligonucléotide 5'CAAC GTTGGGCC3' est perdue ; cette séquence, dénommée Psp1046I ISS, est considérée comme une séquence immunostimulante augmentant les réponses immunitaires de même que la séquence IL-12p40 ISS (Lipford GB et al., 1997, Eur. J. Immunol., 27, 3420-3426).

Une séquence Psp1046 ISS est insérée au site BamHI du plasmide pAG803 par clonage de l'oligonucléotide 25M45 (5'GATCCGGGGGGGAACGTTGGGGGGG3', SEQ ID NO:7) hybridé avec l'oligonucléotide 25M46 (5'GATCCCCCCCCAACGTTCCCCCCCG3', SEQ ID NO:8), pour obtenir le plasmide pAG831.

Une séquence IL-12p40 ISS est insérée au site BamHI du plasmide pAG803 par clonage de l'oligonucléotide 24M63 (5'AGCGCTATGACGTTCCAAGGGCCC3, SEQ ID NO:9) hybridé avec l'oligonucléotide 24M64 (5'GGGCCCTTGGAACGTCATAGCGCT3'. SEQ ID NO:10), pour obtenir le plasmide pAG832.

Après transformation d*'Escherichia coli* de souche XL1 Blue, les plasmides ci-dessus sont amplifiés dans le milieu de culture LB (Sambrook et al., Molecular cloning : A laboratory Manual, Cold Spring Harbor Laboratory Press, Cold Spring Harbor, N.Y., 1989) contenant 25 µg/ml de kanamycine. Après une étape préalable d'élimination de l'endotoxine par traitement des lysats bactériens avec du Triton X-114 (1%), l'ADN plasmidique est purifié sur des colonnes filtres QIA MaxiPrep (QIAGEN) selon les indications du fabricant.

### b) Immunisation des cobayes avec les plasmides pAG831 et pAG832

Des cobayes (Hartley) pesant 300 à 400 g sont immunisés par 2 injections intradermiques dans les flancs avec 50 µg d'ADN des plasmides pAG831 ou pAG832, préparés et purifiés comme indiqué précédemment.

Le témoin est constitué par un groupe de cobayes immunisés par du BCG vivant dans les conditions décrites à l'exemple 1 ou à l'exemple 2.

### c) Mesure de l'activité antigénigue de la protéine Apa produite par des cellules eucaryotes chez les cobayes immunisés par de l'ADN nu, par un test d'hypersensibilité de type retardé

Un et deux mois après l'immunisation, les réactions d'hypersensibilité de type retardé sont mesurées vis-à-vis de la protéine Apa native ou de la protéine Apa recombinante, produite dans une souche de *Escherichia coli* transformée, purifiées selon le protocole décrit dans Horn et al, précité.

L'Apa native et l'Apa recombinante sont injectées en intradermique à la dose de 0,2 µg dans 100 µl de tampon de titrage (tampon D). L'activité antigénique est mesurée comme décrit à l'exemple 2.

### 2. Résultats

Les résultats illustrés par la figure 5 sont les suivants :
- les cobayes immunisés avec le plasmide pAG831 ou pAG832 contenant la séquence codante de l'Apa sous le contrôle d'un promoteur eucaryote développent, dans la très grande majorité des cas, une réponse immunitaire dirigée contre la protéine Apa native (anticorps et une réponse T de type CD4+ mesurable par un test d'hypersensibilité de type retardé ou par un test de prolifération *in vitro* des lymphocytes T lorsqu'ils sont mis en présence des antigènes). Chez les animaux répondant à l'antigène Apa natif, les réponses des lymphocytes T CD4⁺ vis-à-vis de l'antigène déglycosylé par voie enzymatique ou de l'antigène recombinant non-glycosylé provenant de *E. coli* sont de même intensité que les réponses observées avec l'antigène natif glycosylé.
- en revanche, les cobayes immunisés par le BCG vivant montrent une réaction d'hypersensibilité de type retardé uniquement en réponse à l'Apa native. Ces animaux ne développent aucune réaction ou développent une réaction fortement diminuée en réponse à l'Apa recombinante non-glycosylée produite dans *E. coli* comme indiqué précédemment.

Les enseignements de ces résultats sont les suivants :
1) Les résultats observés chez les animaux immunisés avec l'ADN nu codant l'Apa indiquent que la capacité de la protéine Apa à être phagocytée et présentée par les macrophages ou les cellules dendritiques est identique pour la protéine Apa native
   ou recombinante (non glycosylée).
2) La combinaison des résultats ci-dessus avec les résultats observés chez les animaux immunisés avec du BCG vivant indique que l'absence de réponse à la protéine Apa déglycosylée n'est pas due à une diminution de sa capacité à être présentée par les macrophages ou les cellules dendritiques mais à une absence de reconnaissance par les lymphocytes T CD4⁺. En conséquence, les résidus oligomannose des chaînes latérales des protéines Apa ou Lip sous forme native, telles que celles produites par *M. tuberculosis* ou par *BCG* vivant, jouent un rôle dans la constitution d'épitopes T reconnus par des lymphocytes T CD4+.

### EXEMPLE 4 : Préparation des peptides glycosylés SEQ ID NO:1, SEQ ID NO:2 et SEQ ID NO:3.

### 1) Préparation des synthons glycosylés 15, 16 et 19.

Préalablement à la synthèse peptidique, on prépare des synthons glycosylés, c'est-à-dire des thréonines fonctionnalisées par deux ou trois résidus mannoses.

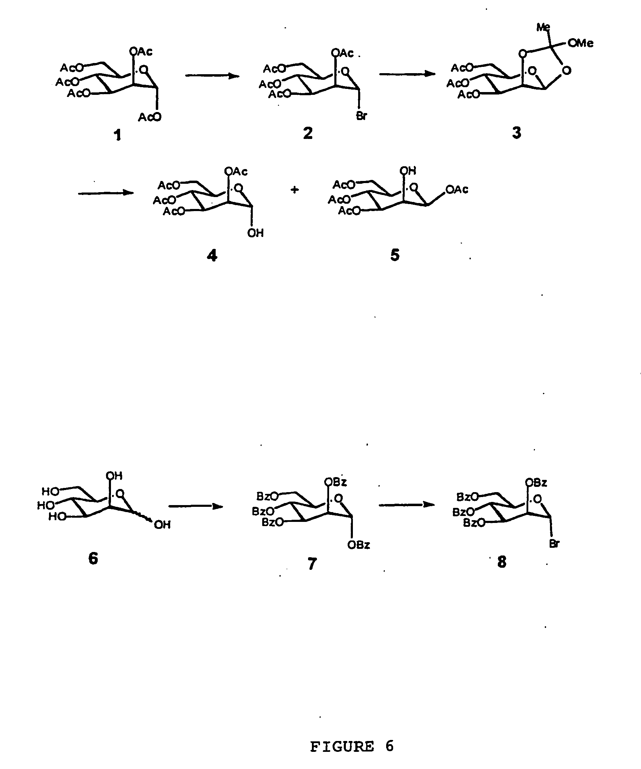

### • Préparation des composés 5 et 8 (figure 6)

La préparation des composés **5** et **8** est décrite, respectivement, par H. FRANZYK et al. dans J. Chem. Soc. Perkin Trans. 1, 1995, 2883-2898 et par R.K. NESS et al. dans J. Am. Chem. Soc. Perkin, 1950, 72, 2200-2205.

Le mannose peracétylé commercial **1** (à savoir le 1,2,3,4,5-penta-O-acétyl-α-D-mannopyranose) est bromé en position anomérique par action du bromure d'hydrogène dans l'acide acétique, comme décrit par A. LEVENE et al. dans J. Biol. Chem., 1931, 90, 89-98. L'intermédiaire 2 activé est cyclisé en orthoester **3** dans un mélange de 2,6-diméthylpyridine/méthanol. L'ouverture régiosélective de l'orthoester par hydrolyse acide, à 0°C, dans un mélange acide trifluoroacétique 10% aqueux/ acétonitrile conduit au 1,3,4,6-tétra-O-acétyl-β-D-mannopyranose (5). Le régioisomère 4 est également isolé.

Le mannose commercial 6 est perbenzoylé en **7** par action du chlorure de benzoyle dans la pyridine. Ce dernier est activé en **8** par action du bromure d'hydrogène dans l'acide acétique. Dans ce protocole et dans ceux qui suivent, d'autres méthodes d'activation que par l'action du bromure d'hydrogène pourraient toutefois être utilisées, telles qu'elles sont connues de l'Homme du métier.

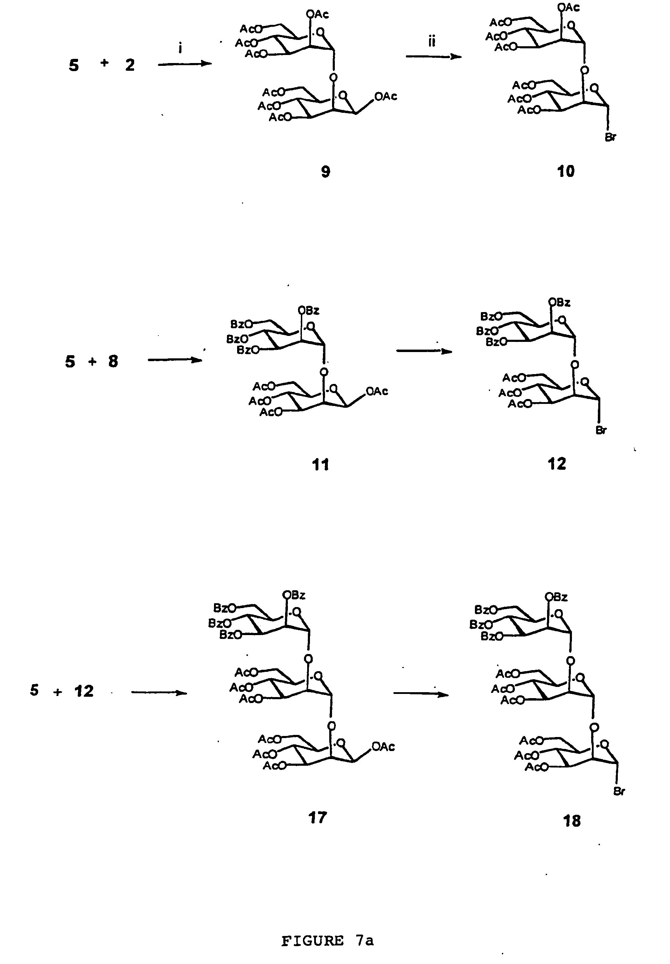

### • Préparation des disaccharides 10 et 12 (figure 7a)

La préparation des composés **10** et **12** est décrite, respectivement, par A. JANSSON et al. dans J. Chem. Soc. Perkin Trans. 1, 1992, 1699-1707 et par H. FRANZYK et al. (ibid*).* Les composés 2 et **5** sont condensés en présence de trifluorométhanesulfonate d'argent (ou tout autre promoteur de la réaction de condensation) dans le dichlorométhane pour conduire au disaccharide peracétylé **9,** qui est ensuite activé en précurseur bromé **10** par action du bromure d'hydrogène dans l'acide acétique. Suivant un protocole identique, les composés **5** et **8** sont condensés pour donner le composé **11,** lui-même activé en **12.**

### • Préparation du trisaccharide 18 (figure 7a)

Le disaccharide activé **12** est condensé sur le monosaccharide accepteur 5, en présence de trifluorométhanesulfonate d'argent dans le dichlorométhane, pour conduire au trisaccharide peracétylé **17,** qui est ensuite activé en précurseur bromé **18** par action du bromure d'hydrogène dans l'acide acétique.

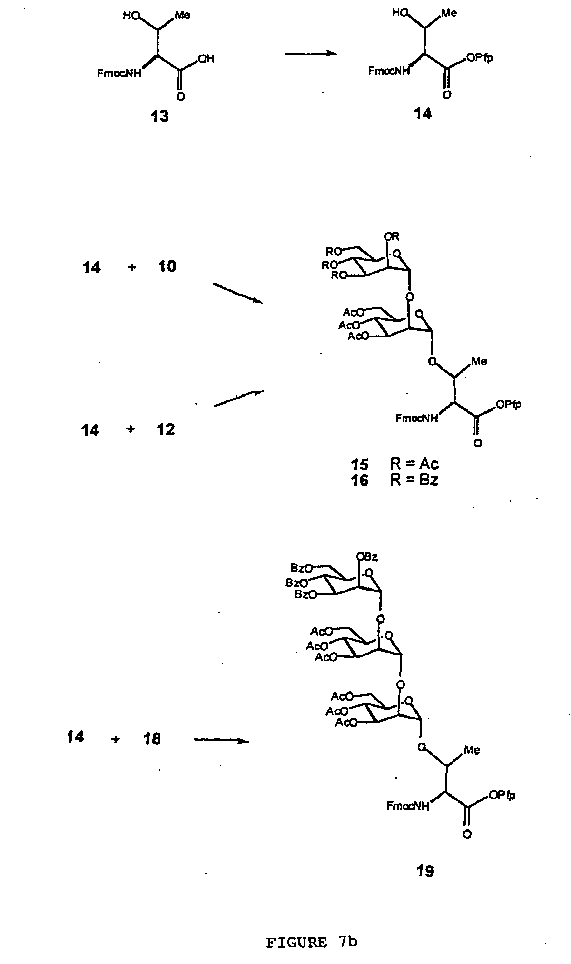

### • Préparation des synthons 15 et 16, porteurs de deux unités mannose, et du synthon 19, porteur de trois unités mannose (figure 7b)

Comme décrit par I. SCHON et al. dans Synthesis, 1986, 303-305, la fonction acide de la thréonine commerciale **13,** dont la fonction amine primaire est protégée par un groupement Fmoc, est bloquée sous forme d'ester par action du pentafluorophénol (pfp) en présence de dicyclohexylcarbodiimide (DCCI) pour conduire au précurseur accepteur **14.**

La préparation des synthons **15** et **16** est décrite, respectivement, par A. JANSSON *et al. (ibid)* et par H. FRANZYK *et al. (ibid).* La condensation du composé **14** avec les disaccharides activés **10** et **12,** menée en présence de trifluorométhanesulfonate d'argent dans le dichlorométhane, conduit aux synthons **15** et **16,** respectivement. Selon le même protocole, la condensation du composé **14** avec le trisaccharide activé **18** conduit au synthon **19.**

### 2) Préparation des peptides glycosylés SEQ ID NO:1, SEQ ID NO:2 et SEQ ID NO:3.

Les peptides sont synthétisés en phase solide en utilisant la chimie Fmoc. La synthèse peptidique est effectuée sur un synthétiseur automatique, à l'aide des acides aminés nécessaires à l'obtention des séquences souhaitées, en incorporant les synthons glycosylés, qui se présentent sous la forme d'esters activés de pentafluorophénol (synthons **15, 16** et **19**).

En fonction des synthons utilisés, on obtient soit des peptides comportant des thréonines fonctionnalisées par deux résidus du mannose (incorporation des synthons **15** et/ou **16** lors de la synthèse peptidique), soit des peptides comportant des thréonines fonctionnalisées par trois résidus du mannose (incorporation du synthon **19** lors de la synthèse peptidique), soit des peptides comportant à la fois des thréonines fonctionnalisées par deux résidus du mannose et des thréonines fonctionnalisées par trois résidus du mannose (incorporation des synthons **19** et **15** et/ou **16** lors de la synthèse peptidique).

En fin de synthèse, après clivage des peptides du support solide à l'aide d'acide trifluoroacétique et déprotection des différents acides aminés et des fonctions hydroxyles des mannoses, les peptides sont purifiés par Chomatographie Liquide Haute Performance (HPLC) en phase inverse. Leur structure est contrôlée par les techniques connues de l'Homme du métier, telles que la spectrométrie de masse et l'analyse en acides aminés.

Les fonctions amides (en position C-terminale des peptides SEQ ID NO:1 et SEQ ID NO:3) et acétates (en position N-terminale des peptides SEQ ID NO:2 et SEQ ID NO:3) sont ensuite introduites par synthèse chimique, au moyen des techniques de chimie organique connues de l'Homme du métier.

### EXEMPLE 5 : Mise en évidence du rôle des résidus oligosaccharidiques de l'Apa dans la définition d'épitopes T par immunisation avec un peptide de l'Apa produit dans E. coli

### 1) Matériel et Méthodes

Un peptide correspondant aux positions 250 à 280 de l'Apa a été produit dans *E. coli,* sous forme d'une fusion avec un fragment de la cyclase de *Bordetella pertussis,* selon les techniques classiques de clonage, d'expression et de purification de protéines recombinantes dans *E.coli* qui sont bien connues de l'Homme du métier (cf par exemple, les protocoles décrits dans Current Protocols in Molecular Biology (Frederick M. AUSUBEL,2000, Wiley and son Inc, Library of Congress, USA).

Trois groupes de 5 cobayes Hartley pesant 300 à 400 g ont été immunisés par 2 injections intradermiques à 1 mois d'intervalle, avec 20 µg de ce peptide de l'Apa purifié, dans 0,1 ml d'une solution d'adjuvant.

Trois groupes de 4 cobayes immunisés quatre mois auparavant par du BCG vivant, dans les conditions décrites à l'exemple 1 sont utilisés comme témoins.

Un et deux mois après l'immunisation, les réactions d'hypersensibilité de type retardé ont été mesurées vis-à-vis de la protéine Apa native, de la protéine Apa recombinante produite dans *E. coli* et de la protéine Apa déglycosylée préparée comme décrit à l'exemple 1, dans les conditions définies à l'exemple 3.

### 2) Résultats

Les réactions d'hypersensibilité de type retardé des cobayes immunisés, soit avec le peptide de fusion de l'Apa, soit avec le BCG vivant ont été mesurées vis-à-vis de la protéine Apa native, de la protéine Apa recombinante produite dans *E. coli* et de la protéine Apa déglycosylée. Les résultats exprimés par le diamètre de la réaction d'érythème (mm) sont présentés dans le Tableau I ci-dessous:

**Tableau I: Activité antigénique du peptide de fusion de l'Apa exprimé dans E.coli**

| **antigène** | **BCG vivant** | **Peptide de fusion** |
|---|---|---|
| Apa native | 17-15-11-13 | 5-12-13-5-5 |
| Apa recombinante *E*. *coli* | 0 0 0 0 | 13-14-15-5-15 |
| Apa déglycosylée | 0 0 0 0 | NT* |

| | | |
|---|---|---|
| *NT: non-testé | | |

Comme indiqué dans le Tableau I ci-dessus, les réactions d'hypersensibilité de type retardé observées chez les cobayes immunisés par le BCG vivant, sont importantes après injection de molécules Apa native. Les réactions sont très faibles ou absentes après injection des molécules déglycosylées par voie chimique ou produites dans *E*. *coli.* A l'inverse pour les cobayes immunisés par les molécules recombinantes, fusion entre le fragment de la cyclase de *Bordetella pertussis* et le fragment interne de la molécule Apa, les sensibilisations sont identiques vis à vis des molécules natives ou déglycosylées.

Ces résultats montrent que les épitopes T glycosylés de la molécule Apa sont reconnus de façon élective par les cobayes immunisés par les bactéries vivantes. Ils montrent aussi que l'absence ou la moindre reconnaissance par ces cobayes des molécules déglycosylées n'est pas liée à une moindre antigénicité intrinsèque de ces molécules

## Revendications

1. Glycopeptide immunogène de 15 à 39 acides aminés issu de *M*. *tuberculosis,* à l'exclusion du glycopeptide de séquence SEQ ID NO:11, lequel glycopeptide comprend un épitope T glycosylé de 14 à 25 acides aminés, parmi lesquels au moins un acide aminé neutre est lié à un di- ou à un trimannose et au moins 15 % d'entre lesdits acides aminés sont des prolines, l'une des prolines étant située en position -1 à -4, relativement à la position dudit acide aminé neutre, et lequel glycopeptide est:
- présenté par une molécule de classe II du CMH,
- reconnu spécifiquement par des lymphocytes T CD4+ induits par immunisation avec la glycoprotéine native dont il est issu, mais n'est pas reconnu par les lymphocytes T CD4+ induits par immunisation avec un peptide non glycosylé de même séquence, et
- capable d'induire une prolifération desdits lymphocytes T CD4+ par lesquels il est reconnu et la sécrétion de cytokines par lesdits lymphocytes.

2. Glycopeptide immunogène selon la revendication 1, **caractérisé en ce que** ledit acide aminé neutre est sélectionné dans le groupe constitué par la sérine et la thréonine.

3. Glycopeptide selon la revendication 1 ou la revendication 2, **caractérisé en ce qu'**il contient 1 à 7 résidus thréonine, liés à un di- ou à un trimannose.

4. Glycopeptide selon l'une quelconque des revendications 1 à 3, **caractérisé en ce que** le di- ou tri-mannose comprend une liaison α-(1,2).

5. Glycopeptide, selon l'une quelconque des revendications 1 à 4, **caractérisé en ce qu'**il est issu de la protéine Apa ou de la protéine Rv1796.

6. Glycopeptide selon l'une quelconque des revendications 1 à 5, **caractérisé en ce qu'**il est sélectionnés dans le groupe constitué par :
- un glycopeptide de 39 acides aminés dont la séquence (SEQ ID NO:1) est celle qui s'étend des positions 1 à 39 de la séquence de la protéine Apa et dans laquelle au moins un des résidus thréonine en position 10, 18 et 27 de la SEQ ID NO:1 est lié à un di- ou tri-mannose par une liaison glycosidique,
- un glycopeptide de 26 acides aminés dont la séquence (SEQ ID NO:2) est celle qui s'étend des positions 261 à 286 de la séquence de la protéine Apa (séquence C-terminale) et dans laquelle le résidu thréonine en position 17 de la SEQ ID NO:2 est lié à un di- ou trimannose par une liaison glycosidique, et
- un glycopeptide de 35 acides aminés dont la séquence (SEQ ID NO:3) est celle qui s'étend des positions 169 à 203 de la séquence de la protéine Rv 1796 et dans laquelle au moins un des résidus thréonine en position 4, 5, 7, 13, 15, 23 et 25 de la SEQ ID NO:3 est lié à un di- ou tri-mannose par une liaison glycosidique.

7. Glycopeptide immunogène issu de *M. tuberculosis,* **caractérisé en ce qu'**il est constitué par l'épitope T glycosylé de 14 à 25 acides aminés tel que défini à l'une quelconque des revendications 1 à 5.

8. Procédé de synthèse d'un glycopeptide selon l'une quelconque des revendications 1 à 7, **caractérisé en ce qu'**il comprend les étapes suivantes :
- préparation, en solution, d'acides aminés neutres glycosylés, liés à un di- ou à un trimannose par une liaison glycosidique,
- synthèse, sur support solide, du glycopeptide issu d'une glycoprotéine de *M. tuberculosis* à l'aide des acides aminés nécessaires à l'obtention de la séquence peptidique dudit glycopeptide et des acides aminés neutres, obtenus précédemment, et
- coupure du glycopeptide du support solide.

9. Procédé selon la revendication 8, **caractérisé en ce que** ledit acide aminé neutre est sélectionné dans le groupe constitué par la sérine et la thréonine.

10. Procédé selon la revendication 9, **caractérisé en ce que** lorsque lesdits glycopeptides présentent les séquences suivantes (**T** représente une thréonine O-glycosylée fonctionnalisée par 2 où 3 résidus mannose et Ac représente une fonction acétate) :
SEQ ID NO:1 : H₂N-DPEPAPPVP**T**TAASPPSTAAAPPAPA**T**PVAPPPPAAANT-CONH₂
SEQ ID NO:2: AcNH-PAPAPAPAGEVAP**T**PTTPTPQRTLPA-COOH
SEQ ID NO:3:
AcNH-TIP**TT**ETPPPPQ**T**V**T**LSPVPPQ**T**V**T**VIPAPPPEEG-CONH₂,
ledit procédé comprend les étapes suivantes :
i) préparation, en solution, de thréonines O-glycosylée fonctionnalisées par 2 ou 3 résidus mannose,
ii) synthèse, sur support solde, des peptides correspondant aux séquences SEQ ID NO:1, SEQ ID NO:2 et SEQ ID NO:3 sus-mentionnées, à l'aide des acides aminés nécessaires à l'obtention de ces séquences et des thréonines O-glycosylées obtenues lors de l'étape i),
iii) coupure des peptides du support solide, et
iv) introduction, par synthèse chimique, d'une fonction amide au niveau de l'extrémité C-terminale des peptides SEQ ID NO:1 et SEQ ID NO:3, et d'une fonction acétate au niveau de l'extrémité N-terminale des peptides SEQ ID NO:2 et SEQ.ID NO:3.

11. Procédé selon la revendication 10, **caractérisé en ce que** les thréonines fonctionnalisées par des résidus mannose sont préparées par les étapes suivantes :
a₂) préparation de dérivés du mannose de formules (I) et (II) : 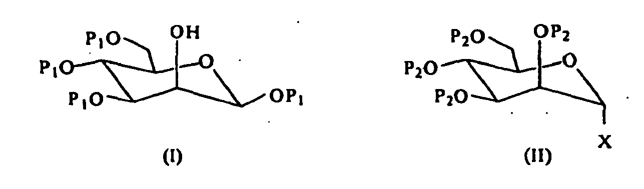 dans lesquelles P₁ et P₂, qui peuvent être identiques ou différents, représentent des groupes protecteurs d'une fonction hydroxyle, et X représente une fonction activée, telle qu'un atome de brome,
b₂) réaction du dérivé de formule (I) avec le dérivé de formule (II), puis activation du composé obtenu, conduisant à l'obtention d'un dérivé activé comportant deux résidus mannose et répondant à la formule (III) : 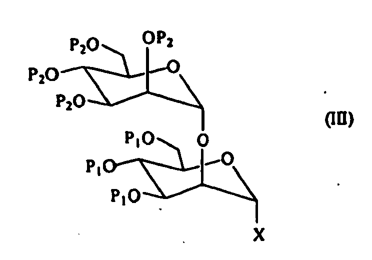 dans laquelle P₁, P₂ et X sont tels que définis en rapport avec les formules (I) et (II),
c₂) éventuellement, réaction du composé de formule (III) avec un dérivé du mannose de formule (I) telle que définie en a₂), puis activation du composé obtenu, conduisant à l'obtention d'un dérivé activé comportant trois résidus mannose et répondant à la formule (IV) : 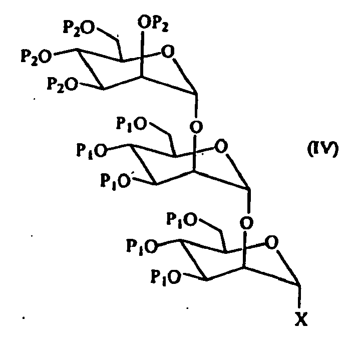 dans laquelle P₁, P₂ et X sont tels que définis en rapport avec les formules (I) et (II), et
d₂) condensation du composé de formule (III) ou du composé de formule (IV) avec une thréonine convenablement protégée de formule (V) : 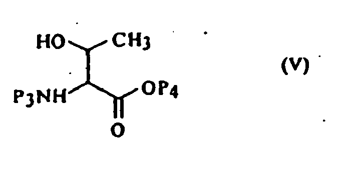 dans laquelle P₃ représente un groupe protecteur d'une fonction amine primaire et P₄ représente un groupe protecteur d'une fonction carboxyle,
conduisant respectivement à l'obtention d'une thréonine glycosylée de formule (VI) ou (VII) : 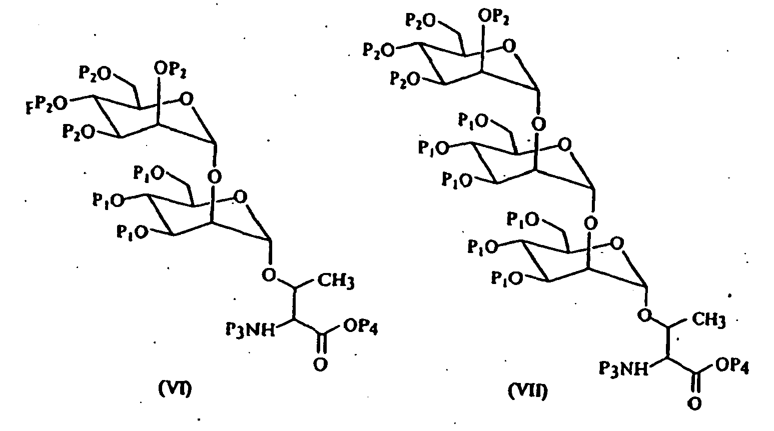 dans lesquelles P₁, P₂, P₃ et P₄ sont tels que définis précédemment.

12. Procédé de sélection et de criblage de glycopeptides antigéniques à partir de la séquence peptidique de glycoprotéines de *M*. *tuberculosis,* **caractérisé en ce qu'**il comprend au moins les étapes suivantes :
a₃) recherche et sélection dans la séquence peptidique desdites glycoprotéines d'au moins une séquence de 14 à 25 acides aminés, contenant au moins un acide aminé neutre lié à un di- ou un trimannose et au moins 15 % de proline, l'une des proline étant située en position -1 à -4, relativement à la position dudit acide aminé;
b₃) préparation du/des glycopeptide(s) sélectionné(s) à l'étape a₃) conformément au procédé selon les revendications 8 à 11, et
c₃) sélection des glycopeptides dont l'activité antigénique est au moins 10 fois supérieure, de préférence au moins 30 fois supérieure, à celle d'un peptide témoin de même séquence.

13. Procédé selon la revendication 12, **caractérisé en ce que**, préalablement à l'étape a₃), il comprend une étape de présélection d'au moins une glycoprotéine antigénique.

14. Procédé selon la revendication 12, **caractérisé en ce que** ledit acide aminé neutre est sélectionné dans le groupe constitué par la sérine et la thréonine.

15. Procédé selon la revendication 12, **caractérisé en ce que**, à l'étape c₃), l'activité antigénique dudit glycopeptide est évaluée par mesure de l'activation des lymphocytes T CD4+ d'animaux immunisés par *Mycobacterium bovis* BCG ou par une fraction antigénique de *Mycobacterium tuberculosis.*

16. Glycopeptide tel que défini à l'une quelconque des revendications 1 à 7, **caractérisé en ce qu'**il est susceptible d'être obtenu par le procédé de sélection selon l'une quelconque des revendications 12 à 15 ou le procédé de synthèse selon l'une quelconque des revendications 8 à 11.

17. Composition immunogène apte à induire une immunité humorale et/ou cellulaire, **caractérisée en ce qu'**elle comprend au moins un glycopeptide selon l'une quelconque des revendications 1 à 7 ou 16 ou un glycopeptide de séquence SEQ ID NO:11, associé à au moins un véhicule pharmaceutiquement acceptable.

18. Composition vaccinale apte à induire une immunité humorale et/ou cellulaire, **caractérisée en ce qu'**elle comprend au moins un glycopeptide selon l'une quelconque des revendications 1 à 7 ou 16 ou un glycopeptide de séquence SEQ ID NO:11, associé à au moins un véhicule pharmaceutiquement acceptable et éventuellement à au moins un adjuvant.

19. Composition immunogène ou vaccinale selon la revendication 17 ou la revendication 18, **caractérisée en ce que** ledit glycopeptide est associé à une protéines ou un fragment de protéine comprenant au moins un épitope B, un épitope T de type CD4+ ou un épitope T de type CD8+.

20. Anticorps, **caractérisé en ce qu'**il est dirigé spécifiquement contre un ou plusieurs des glycopeptides selon l'une quelconque des revendications 1 à 7 ou 16.

21. Réactif de diagnostic, **caractérisé en ce qu'**il comprend un glycopeptide selon l'une quelconque des revendications 1 à 7 ou 16, un glycopeptide de séquence SEQ ID NO:11 ou un anticorps selon la revendication 20.

22. Procédé de détection de la tuberculose, **caractérisé en qu'**il comprend la mise en contact d'un échantillon biologique d'un patient susceptible d'être infecté par *Mycobacterium tuberculosis,* avec un réactif de diagnostic selon la revendication 21 et la détection de la formation d'un complexe anticorps-microorganisme présent dans l'échantillon biologique ou glycopeptide(s)-anticorps présents dans l'échantillon.

23. Procédé de détection de la tuberculose, **caractérisé en qu'**il comprend la détection, *in vitro,* de lymphocytes T CD4+ reconnaissant un glycopeptide selon l'une quelconque des revendications 1 à 7 ou 16, par toute technique appropriée.

24. Procédé selon la revendication 23, **caractérisé en ce que** ladite technique de détection est sélectionnée dans le groupe constitué par les tests de prolifération de lymphocytes T et les dosages immunoenzymatiques de cytokines spécifiques de lymphocytes T CD4+.

## Claims

1. Immunogenic glycopeptide of 15 to 39 amino acids derived from *M tuberculosis,* excluding the glycopeptide of sequence SEQ ID NO: 11, said glycopeptide comprising a glycosylated T epitope comprising from 14 to 25 amino acids, among which at least one neutral amino acid is bonded to a di- or trimannose and at least 15% of said amino acids are prolines, one of the prolines being situated in position -1 to -4, relative to the position of said neutral amino acid, and said glycopeptides is:
- presented by a class II MHC molecule,
- specifically recognised by T CD4+ lymphocytes induced by immunisation with the native glycoprotein from which it is derived, but is not recognised by the T CD4+ lymphocytes induced by immunisation with a non-glycosylated peptide of the same sequence, and
- capable of inducing a proliferation of said T CD4+ lymphocytes by which it is recognised and the secretion of cytokines by said lymphocytes.

2. Immunogenic glycopeptide according to claim 1, **characterised in that** said neutral amino acid is selected from the group consisting of serine and threonine.

3. Glycopeptide according to claim 1 or claim 2, **characterised in that** it contains 1 to 7 threonine residues, bonded to a di- or trimannose.

4. Glycopeptide according to any one of claims 1 to 3, **characterised in that** the di- or trimannose comprises an α-(1,2) bond.

5. Glycopeptide, according to any one of claims 1 to 4, **characterised in that** it is derived from the Apa protein or the Rv1796 protein.

6. Glycopeptide according to any one of claims 1 to 5, **characterised in that** it is selected from the group consisting of:
- a glycopeptide of 39 amino acids whose sequence (SEQ ID NO: 1) is that extending from positions 1 to 39 of the Apa protein sequence and in which at least one of the threonine residues in position 10, 18 and 27 of the SEQ ID NO:1 is bonded to a di- or tri-mannose via glycosidic bond,
- a glycopeptide of 26 amino acids whose sequence (SEQ ID NO:2) is that extending from positions 261 to 286 of the Apa protein sequence (C-terminal sequence) and in which the threonine residues in position 17 of the SEQ ID NO:2 is bonded to a di- or tri-mannose via a glycosidic bond, and
- a glycopeptide of 35 amino acids whose sequence (SEQ ID NO:3) is that extending from positions 169 to 203 of the Rv 1796 protein sequence and in which at least one of the threonine residues in position 4, 5, 7, 13, 15, 23 and 25 of the SEQ ID NO:3 is bonded to a di- or tri-mannose via a glycosidic bond.

7. Immunogenic glycopeptide derived from *M. tuberculosis,* **characterised in that** it consists of the glycosylated T epitope comprising from 14 to 25 amino acids such as defined in any one of claims 1 to 5.

8. Process for synthesising a glycopeptide according to any one of claims 1 to 7, **characterised in that** it comprises the following steps:
- preparation, in solution, of glycosylated neutral amino acids, bonded to a di- or trimannose via a glycosidic bond,
- synthesis, on a solid support, of the glycopeptide derived from a glycoprotein of *M. tuberculosis* using the amino acids necessary for obtaining the peptidic sequence of said glycopeptide and neutral amino acids, previously obtained,
- cleaving of the glycopeptide from the solid support.

9. Process according to claim 8, **characterised in that** said neutral amino acid is selected from the group consisting of serine and threonine.

10. Process according to claim 9, **characterised in that** when said glycopeptides present the following sequences (**T** represents an O-glycosylated threonine functionalised with 2 or 3 mannose residues and Ac represents an acetate function):
SEQ ID NO:1:
H₂N-DPEPAPPVP**T**TAASPPS**T**AAAPPAPA**T**PVAPPPPAAANT-CONH₂
SEQ ID NO:2:
AcNH-PAPAPAPAGEVAPTPT**T**PTPQRTLPA-COOH
SEQ ID NO:3:
AcNH-TIP**TT**E**T**PPPPQ**T**V**T**LSPVPPQ**T**V**T**VIPAPPPEEG-CONH2,
said process comprising the following steps:
i) preparation, in solution, of O-glycosylated threonines functionalised with 2 or 3 mannose residues,
ii) synthesis, on a solid support, of the peptides corresponding to the abovementioned sequences SEQ ID NO:1, SEQ ID NO:2 and SEQ ID NO:3, using the amino acids necessary for obtaining these sequences and the O-glycosylated threonines obtained in step i),
iii) cleaving of the peptides from the solid support, and
iv) introduction, by chemical synthesis, of an amide function at the level of the C-terminal end of the peptides SEQ ID NO:1 and SEQ ID NO:3, and of an acetate function at the level of the N-terminal end of the peptides SEQ ID NO:2 and SEQ ID NO:3.

11. Process according to claim 10, **characterised in that** the threonines functionalised with mannose residues are prepared by means of the following steps:
a₂) preparation of mannose derivatives of formulas (I) and (II): 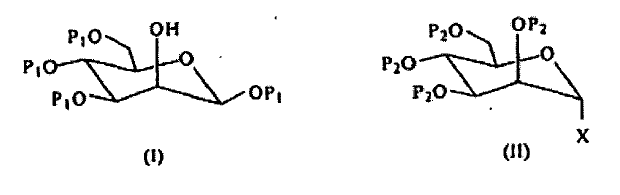 in which P₁ and P₂, which may be identical or different, represent protecting groups for a hydroxyl function, and X represents an activated function, such as a bromine atom,
b₂) reaction of the derivative of formula (I) with the derivative of formula (II), followed by activation of the compound obtained, leading to the obtaining of an activated derivative comprising two mannose residues and corresponding to formula (III): 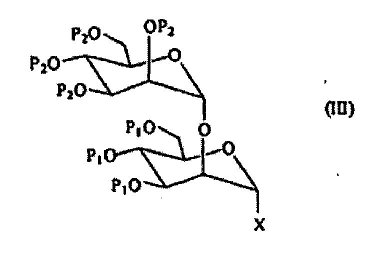 in which P₁, P₂ and X are as defined in relation to formulas (I) and (II),
c₂) if necessary, reaction of the compound of formula (III) with a mannose derivative of formula (I) as defined in a₂), followed by activation of the compound obtained, leading to the obtaining of an activated derivative comprising three mannose residues and corresponding to formula (IV): 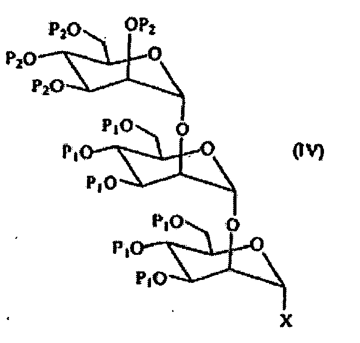 in which P₁, P₂ and X are as defined in relation to formulas (I) and (II), and
d₂) condensation of the compound of formula (III) or of the compound of formula (IV) with an appropriately protected threonine of formula (V): 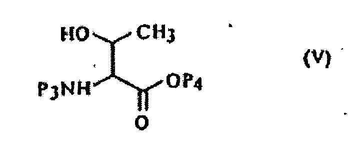 in which P₃ represents a protecting group for a primary amine function and P₄ represents a protecting group for a carboxyl function,
leading respectively to the obtaining of a glycosylated threonine of formula (VI) or (VII): 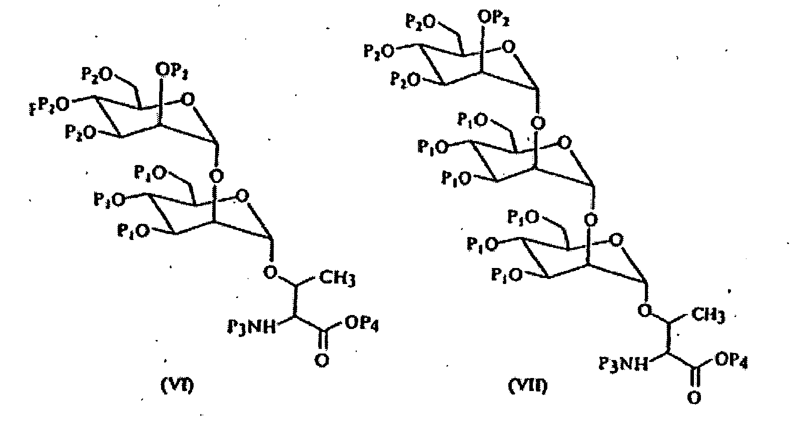 in which P₁, P₂, P₃ and P₄ are as defined above.

12. Process of selection and screening of antigenic glycopeptides based on the peptidic sequence of glycoproteins of *M. tuberculosis,* **characterised in that** it comprises at least the following steps:
a₃) searching for and selecting within the peptide sequence of said glycoproteins, at least one 14 to 25 amino acids sequence, containing at least one neutral amino acid bonded to a di- or trimannose and at least 15% proline, one of the prolines being situated in position -1 to -4, relative to the position of said amino acid,
b₃) preparation of the glycopeptide(s) selected in step a₃) in accordance with the process according to claims 8 to 11, and
c₃) selection of the glycopeptides whose antigenic activity is at least 10 times greater, preferably 30 times greater, than that of a control peptide of the same sequence.

13. Process according to claim 12, **characterised in that**, prior to step a₃), it comprises a step of pre-selection of at least on antigenic glycoprotein.

14. Process according to claim 12, **characterised in that** said neutral amino acid is selected from the group consisting of serine and threonine.

15. Process according to claim 12, **characterised in that**, in step c₃), the antigenic activity of said glycopeptide is evaluated by measuring the activation of the T CD4+ lymphocytes of animals immunised with *Mycobacterium bovis* BCG or by an antigenic fraction of *Mycobacterium tuberculosis.*

16. Glycopeptide as defined in any one of claims 1 to 7, **characterised in that** it can be obtained by the selection process according to any one of claims 12 to 15 or the synthesising process according to any one of claims 8 to 11.

17. Immunogenic composition capable of inducing humoral and/or cellular immunity, **characterised in that** it comprises at least one glycopeptide according to any one of claims 1 to 7 or 16 or a glycopeptide of sequence SEQ ID NO: 11, associated with at least one pharmaceutically acceptable vehicle.

18. Vaccine composition capable of inducing humoral and/or cellular immunity, **characterised in that** it comprises at least one glycopeptide according to any one of claims 1 to 7 or 16 or a glycopeptide of sequence SEQ ID NO: 11, associated with at least one pharmaceutically acceptable vehicle and, optionnally, with at least one adjuvant.

19. Immunogenic or vaccine composition according to claim 17 or claim 18, **characterised in that** said glycopeptide is associated with a protein or a protein fragment comprising at least a B epitope, a T epitope of the CD4+ type or a T epitope of the CD8+ type.

20. Antibody, **characterised in that** it is directed specifically against one or more of the glycopeptides according to any one of claims 1 to 7 or 16.

21. Diagnostic reagent, **characterised in that** it comprises a glycopeptide according to any one of claims 1 to 7 or 16, a glycopeptide of sequence SEQ ID NO:11 or an antibody according to claim 20.

22. Process for detecting tuberculosis, **characterised in that** it comprises bringing of a biological sample from a patient likely to be infected with *Myobacterium tuberculosis* into contact with a diagnostic reagent according to claim 21 and detecting the formation of an antibody-microorganism present in the biological sample complex or glycopeptide(s)-antibodies present in the sample complex.

23. Process for detecting tuberculosis, **characterised in that** it comprises the *in vitro* detection of T CD4+ lymphocytes recognising a glycopeptide according to any one of claims 1 to 7 or 16, by means of any appropriate technique.

24. Process according to claim 23, **characterised in that** said detection technique is selected from the group consisting of the T lymphocyte proliferation assays and the immunoenzymatic dosages of T CD4+ lymphocytes specific cytokines.

## Patentansprüche

1. Immunogenes Glykopeptid von 15 bis 39 Aminosäuren, das von *M. tuberculosis* stammt, mit Ausnahme des Glykopeptids der Sequenz SEQ ID NO: 11, wobei das Glykopeptid ein glykosyliertes T-Epitop von 14 bis 25 Aminosäuren umfasst, unter denen wenigstens eine neutrale Aminosäure an eine Di- oder Trimannose gebunden ist und wenigstens 15% unter den genannten Aminosäuren Proline sind, wobei eines der Proline in Position -1 bis -4, bezogen auf die Position der genannten neutralen Aminosäure, angeordnet ist und wobei das Glykopeptid:
- von einem Molekül der MHC-Klasse-II präsentiert wird,
- spezifisch erkannt wird durch (CD4+)-T-Lymphozyten, induziert durch Immunisierung mit dem nativen Glykoprotein, von dem es abstammt, aber nicht erkannt wird durch (CD4+)-T-Lymphozyten, induziert durch Immunisierung mit einem nichtglykosylierten Peptid der gleichen Sequenz, und
- befähigt ist, eine Proliferation der genannten (CD4+)-T-Lymphozyten, durch die es erkannt wird, und die Sekretion von Cytokinen durch die genannten Lymphozyten zu induzieren.

2. Immunogenes Glykopeptid gemäß Anspruch 1, **dadurch gekennzeichnet, dass** die genannte neutrale Aminosäure ausgewählt ist aus der Gruppe, die von Serin und Threonin gebildet wird.

3. Glykopeptid gemäß Anspruch 1 oder Anspruch 2, **dadurch gekennzeichnet, dass** es 1 bis 7 Threoninreste, gebunden an eine Di- oder an eine Trimannose, enthält.

4. Glykopeptid gemäß einem der Ansprüche 1 bis 3, **dadurch gekennzeichnet, dass** die Di- oder Trimannose eine α-(1,2)-Bindung umfasst.

5. Glykopeptid gemäß einem der Ansprüche 1 bis 4, **dadurch gekennzeichnet, dass** es von dem Protein Apa oder von dem Protein Rv796 abstammt.

6. Glykopeptid gemäß einem der Ansprüche 1 bis 5, **dadurch gekennzeichnet, dass** es ausgewählt ist aus der Gruppe, gebildet von:
- einem Glykopeptid von 39 Aminosäuren, dessen Sequenz (SEQ ID NO: 1) die ist, die sich über die Positionen 1 bis 39 der Sequenz des Proteins Apa erstreckt und in der wenigstens einer der Threoninreste in Position 10, 18 und 27 der SEQ ID NO: 1 über eine glykosidische Bindung an eine Di- oder Trimannose gebunden ist,
- einem Glykopeptid von 26 Aminosäuren, dessen Sequenz (SEQ ID NO: 2) die ist, die sich über die Positionen 261 bis 286 der Sequenz des Proteins Apa (C-terminale Sequenz) erstreckt und in der der Threoninrest in Position 17 der SEQ ID NO: 2 über eine glykosidische Bindung an eine Di- oder Trimannose gebunden ist, und
- einem Glykopeptid von 35 Aminosäuren, dessen Sequenz (SEQ ID NO: 3) die ist, die sich über die Positionen 169 bis 203 der Sequenz des Proteins Rv1796 erstreckt und in der wenigstens einer der Threoninreste in Position 4, 5, 7, 13, 15, 23 und 25 der SEQ ID NO: 3 über eine glykosidische Bindung an eine Di- oder Trimannose gebunden ist.

7. Immunogenes Glykopeptid, das von *M. tuberculosis* stammt, **dadurch gekennzeichnet, dass** es durch das glykosylierte T-Epitop von 14 bis 25 Aminosäuren, wie es in einem der Ansprüche 1 bis 5 definiert ist, gebildet ist.

8. Verfahren zur Synthese eines Glykopeptids gemäß einem der Ansprüche 1 bis 7, **dadurch gekennzeichnet, dass** es die folgenden Schritte umfasst:
- Herstellung von glykosylierten neutralen Aminosäuren, die über eine glykosidische Bindung an eine Di- oder Trimannose gebunden sind, in Lösung,
- Synthese des Glykopeptids, das von einem Glykoprotein von *M. tuberculosis* abstammt, auf festem Träger mit Hilfe der Aminosäuren, die zum Erhalten der Peptidsequenz des genannten Glykopeptids nötig sind, und der neutralen Aminosäuren, die zuvor erhalten wurden, und
- Abtrennung des Glykopeptids vom festen Träger.

9. Verfahren gemäß Anspruch 8, **dadurch gekennzeichnet, dass** die neutrale Aminosäure ausgewählt ist aus der Gruppe, die von Serin und Threonin gebildet wird.

10. Verfahren gemäß Anspruch 9, **dadurch gekennzeichnet, dass**, wenn die genannten Glykopeptide die folgenden Sequenzen darstellen (T stellt ein O-glykosyliertes Threonin, das durch zwei oder drei Mannosereste funktionalisiert ist, dar und Ac stellt eine Acetatfunktion dar):
SEQ ID NO:1 :
H₂N-DPEPAPPVPT**T**AASPPS**T**AAAPPAPA**T**PVAPPPPAAANT-CONH₂
SEQ ID NO:2:
AcNH-PAPAPAPAGEVAPTPT**TP**TPQRTLPA-COOH
SEQ ID NO:3 :
AcNH-TIP**TT**E**T**PPPPQ**T**V**T**LSPVPPQ**T**V**T**VIPAPPPEEG-CONH₂,
das genannte Verfahren die folgenden Schritte umfasst:
i) Herstellung von O-glykosylierten Threoninen, die durch zwei oder drei Mannosereste funktionalisiert sind, in Lösung,
ii) Synthese von Peptiden, die den oben angegebenen Sequenzen SEQ ID NO: 1, SEQ ID NO: 2 und SEQ ID NO: 3 entsprechen, auf festem Träger mit Hilfe der Aminosäuren, die zum Erhalten dieser Sequenzen nötig sind und den O-glykosylierten Threoninen, die im Schritt i) erhalten wurden,
iii)Abtrennung der Peptide vom festen Träger und
iv) Einführung einer Amidfunktion am C-terminalen Ende der Peptide SEQ ID NO: 1 und SEQ ID NO: 3 und einer Acetatfunktion am N-terminalen Ende der Peptide SEQ ID NO: 2 und SEQ ID NO: 3 mittels chemischer Synthese.

11. Verfahren gemäß Anspruch 10, **dadurch gekennzeichnet, dass** die durch Mannosereste funktionalisierten Threonine mittels den folgenden Schritten hergestellt werden:
a₂) Herstellung von Mannose-Derivaten der Formeln (I) und (II) : 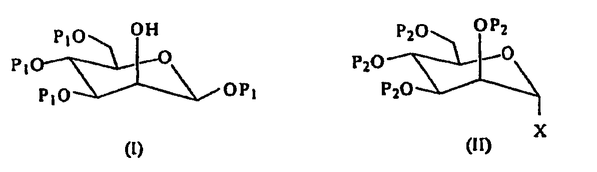
in denen P₁ und P₂, die identisch oder verschieden sein können, Schutzgruppen einer Hydroxylfunktion darstellen und X eine aktivierte Funktion, wie ein Bromatom, darstellt,
b₂) Umsetzung des Derivats der Formel (I) mit dem Derivat der Formel (II), dann Aktivierung der erhaltenen Verbindung, was zum Erhalt eines aktivierten Derivats führt, das zwei Mannosereste aufweist und der Formel (III) entspricht: 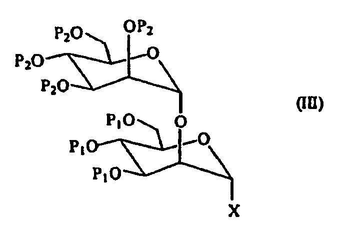
in der P₁, P₂ und X sind, wie in Bezug auf die Formeln (I) und (II) definiert,
c₂) gegebenenfalls Umsetzung der Verbindung der Formel (III) mit einem Mannose-Derivat der Formel (I), wie sie in a₂) definiert ist, dann Aktivierung der erhaltenen Verbindung, was zum Erhalt eines aktiven Derivats führt, das drei Mannosereste aufweist und der Formel (IV) entspricht: 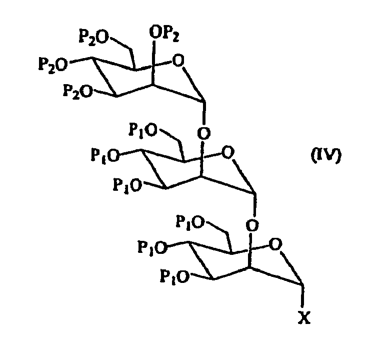
in der P₁, P₂ und X sind, wie in Bezug auf die Formeln (I) und (II) definiert, und
d₂) Kondensation der Verbindung der Formel (III) oder der Verbindung der Formel (IV) mit einem zweckdienlicherweise geschützten Threonin der Formel (V): 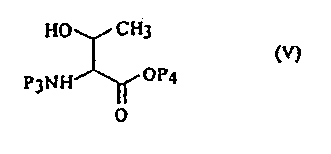 in der P₃ eine Schutzgruppe einer primären Aminfunktion darstellt und P₄ eine Schutzgruppe einer Carboxylfunktion darstellt,
was zum Erhalt eines glykosylierten Threonins der Formel (VI) beziehungsweise (VII) führt: 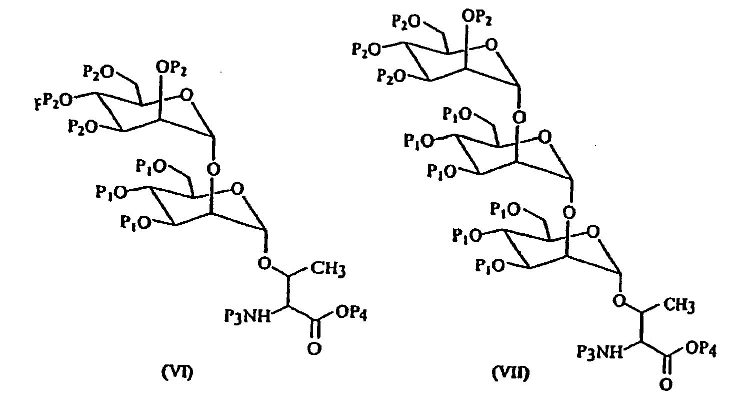 in denen P₁, P₂, P₃ und P₄ sind, wie zuvor definiert.

12. Verfahren zur Auswahl und zur Durchmusterung von antigenen Glykopeptiden ausgehend von der Peptidsequenz von Glykoproteinen von *M. tuberculosis,* **dadurch gekennzeichnet, dass** es wenigstens die folgenden Schritte umfasst:
a₃) Suche und Auswahl in der Peptidsequenz der genannten Glykoproteine nach bzw. von wenigstens einer Sequenz von 14 bis 25 Aminosäuren, die wenigstens eine neutrale Aminosäure, die an eine Di- oder Trimannose gebunden ist, und wenigstens 15% an Prolin enthält, wobei wenigstens eines der Proline in Position -1 bis -4, bezogen auf die Position der genannten Aminosäure, angeordnet ist,
b₃) Herstellung des/der Glykopeptids/Glykopeptide, das/die in Schritt a₃) ausgewählt wurde(n), nach dem Verfahren gemäß den Ansprüchen 8-11 und
c₃) Auswahl von Glykopeptiden, deren antigene Aktivität wenigstens 10-fach über, vorzugsweise wenigstens 30-fach über der eines Kontrollpeptids der gleichen Sequenz ist.

13. Verfahren gemäß Anspruch 12, **dadurch gekennzeichnet, dass** es vor dem Schritt a₃) einen Schritt der Vorauswahl von wenigstens einem antigenen Glykoprotein umfasst.

14. Verfahren gemäß Anspruch 12, **dadurch gekennzeichnet, dass** die genannte neutrale Aminosäure ausgewählt ist aus der Gruppe, die von Serin und Threonin gebildet wird.

15. Verfahren gemäß Anspruch 12, **dadurch gekennzeichnet, dass** die antigene Aktivität des genannten Glykopeptids im Schritt c₃) mittels der Aktivierung von (CD4+)-T-Lymphozyten von Tieren, die mit *Mycobacterium bovis* BCG oder mit einer antigenen Fraktion von *Mycobacterium tuberculosis* immunisiert wurden, bewertet wird.

16. Glykopeptid, wie es in einem der Ansprüche 1 bis 7 definiert ist, **dadurch gekennzeichnet, dass** es durch das Verfahren zur Auswahl gemäß einem der Ansprüche 12 bis 15 oder dem Verfahren zur Synthese gemäß einem der Ansprüche 8 bis 11 erhalten werden kann.

17. Immunogene Zusammensetzung, die dazu befähigt ist, eine humorale und/oder zelluläre Immunität zu induzieren, **dadurch gekennzeichnet, dass** sie wenigstens ein Glykopeptid gemäß einem der Ansprüche 1 bis 7 oder 16 oder ein Glykopeptid der Sequenz SEQ ID NO: 11, in Verbindung mit wenigstens einem pharmazeutisch verträglichen Vehikel, umfasst.

18. Vakzinzusammensetzung, die dazu befähigt ist, eine humorale und/oder zelluläre Immunität zu induzieren, **dadurch gekennzeichnet, dass** sie wenigstens ein Glykopeptid gemäß einem der Ansprüche 1 bis 7 oder 16 oder ein Glykopeptid der Sequenz SEQ ID NO: 11, in Verbindung mit wenigstens einem pharmazeutisch verträglichen Vehikel und gegebenenfalls wenigstens einem Adjuvans, umfasst.

19. Immunogene oder Vakzinzusammensetzung gemäß Anspruch 17 oder Anspruch 18, **dadurch gekennzeichnet, dass** das genannte Glykopeptid mit einem Protein oder einem Proteinfragment, das wenigstens ein B-Epitop, ein T-Epitop vom Typ CD4+ oder ein T-Epitop vom Typ CD8+ umfasst, verbunden ist.

20. Antikörper, **dadurch gekennzeichnet, dass** er spezifisch gegen eines oder mehrere der Glykopeptide gemäß einem der Ansprüche 1 bis 7 oder 16 gerichtet ist.

21. Diagnosereagenz, **dadurch gekennzeichnet, dass** es ein Glykopeptid gemäß einem der Ansprüche 1 bis 7 oder 16, ein Glykopeptid der Sequenz SEQ ID NO: 11 oder einen Antikörper gemäß Anspruch 20 umfasst.

22. Verfahren zur Detektion der Tuberkulose, **dadurch gekennzeichnet, dass** es das Inkontaktbringen einer biologischen Probe eines Patienten, der dafür anfällig ist, von *Mycobacterium tuberculosis* infiziert zu sein bzw. zu werden, mit einem Diagnosereagenz gemäß Anspruch 21 und die Detektion der Bildung eines Komplexes Antikörper-Mikroorganismus, der in der biologischen Probe vorhanden ist, oder Glykopeptid(e)-Antikörper, die in der Probe vorhanden sind, umfasst.

23. Verfahren zur Detektion der Tuberkulose, **dadurch gekennzeichnet, dass** es die in vitro-Detektion von (CD4+)-T-Lymphozyten, die ein Glykopeptid gemäß einem der Ansprüche 1 bis 7 oder 16 erkennen, mittels jeder geeigneten Technik umfasst.

24. Verfahren gemäß Anspruch 23, **dadurch gekennzeichnet, dass** die genannte Technik zur Detektion ausgewählt ist aus der Gruppe, die von den Tests der Proliferation von T-Lymphozyten und den immunoenzymatischen Bestimmungen von Cytokinen, die für (CD4+)-T-Lymphozyten spezifisch sind, gebildet wird.


### LISTE DE SEQUENCES

<110> INSTITUT PASTEUR
   Marchal Gilles
   Romain Félix
   Pescher Pascale
<120> Glycopeptides immunogènes, criblage, préparation et
   applications
<130> Seq226FR86
<140>
   <141>
<160> 11
<170> PatentIn Ver. 2.1
<210> 1
   <211> 39
   <212> PRT
   <213> Mycobacterium tuberculosis
<220>
   <221> MOD_RES
   <222> (10)
   <223> liaison glycosidique à un di- ou un tri-saccharide
<220>
   <221> MOD_RES
   <222> (18)
   <223> liaison glycosidique à un di-ou à un tri-saccharide
<220>
   <221> MOD_RES
   <222> (27)
   <223> liaison glycosidique à un di-ou à un tri-saccharide
<400> 1
   Asp Pro Glu Pro Ala Pro Pro Val Pro Thr Thr Ala Ala Ser Pro Pro
   1 5 10 15
   Ser Thr Ala Ala Ala Pro Pro Ala Pro Ala Thr Pro Val Ala Pro Pro
   20 25 30
   Pro Pro Ala Ala Ala Asn Thr
   35
<210> 2
   <211> 26
   <212> PRT
   <213> Mycobacterium tuberculosis
<220>
   <221> MOD_RES
   <222> (17)
<223> liaison glycosidique à un di- ou à un
   tri-saccharide
<400> 2
   Pro Ala Pro Ala Pro Ala Pro Ala Gly Glu Val Ala Pro Thr Pro Thr
   1 5 10 15
   Thr Pro Thr Pro Gln Arg Thr Leu Pro Ala
   20 25
<210> 3
   <211> 35
   <212> PRT
   <213> Mycobacterium tuberculosis
<220>
   <221> MOD_RES
   <222> (4)
   <223> liaison glycosidique à un di- ou à un tri-saccharide
<220>
   <221> MOD_RES
   <222> (5)
   <223> liaison glycosidique à un di- ou à un tri-saccharide
<220>
   <221> MOD_RES
   <222> (7)
   <223> liaison glycosidique à un di- ou à un tri-saccharide
<220>
   <221> MOD_RES
   <222> (13)
   <223> liaison glycosidique à un di- ou à un tri-saccharide
<220>
   <221> MOD_RES
   <222> (15)
   <223> liaison glycosidique à un di- ou à un tri-saccharide
<220>
   <221> MOD_RES
   <222> (23)
   <223> liaison glycosidique à un di- ou à un tri-saccharide
<220>
   <221> MOD_RES
   <222> (25)
   <223> liaison glycosidique à un di- ou à un
   tri-saccharide
<400> 3
   Thr Ile Pro Thr Thr Glu Thr Pro Pro Pro Pro Gln Thr Val Thr Leu
   1 5 10 15
   Ser Pro Val Pro Pro Gln Thr Val Thr Val Ile Pro Ala Pro Pro Pro
   20 25 30
   Glu Glu Gly
   35
<210> 4
   <211> 12
   <212> ADN
   <213> Séquence artificielle
<220>
   <221> MOD_RES
   <222> (29)
   <223> liaison glycosidique à un mannose, un di-mannose
   ou un tri-mannose
<400> 4
   caacgttggg cc 12
<210> 5
   <211> 20
   <212> ADN
   <213> Séquence artificielle
<220>
   <223> Description de la séquence artificielle:amorce
<400> 5
   tcccaagctt ttggtagccg 20
<210> 6
   <211> 33
   <212> ADN
   <213> Séquence artificielle
<220>
   <223> Description de la séquence artificielle:linker
<400> 6
   ctaggatcca ccatgccgga gccagcgccc ccg 33
<210> 7
   <211> 25
   <212> ADN
   <213> Séquence artificielle
<220>
   <223> Description de la séquence artificielle:linker
<400> 7
   gatccggggg ggaacgttgg ggggg 25
<210> 8
   <211> 25
   <212> ADN
   <213> Séquence artificielle
<220>
   <223> Description de la séquence artificielle:linker
<400> 8
   gatccccccc caacgttccc ccccg 25
<210> 9
   <211> 24
   <212> ADN
   <213> Séquence artificielle
<220>
   <223> Description de la séquence artificielle:linker
<400> 9
   agcgctatga cgttccaagg gccc 24
<210> 10
   <211> 24
   <212> ADN
   <213> Séquence artificielle
<220>
   <223> Description de la séquence artificielle:linker
<400> 10
   gggcccttgg aacgtcatag cgct 24
<210> 11
   <211> 35
   <212> PRT
   <213> Mycobacterium tuberculosis
<220>
   <221> MOD_RES
   <222> (29)
   <223> liaison glycosidique à un mannose, un di-mannose
   ou un tri-mannose
<400> 11
   Val Ala Pro Pro Pro Ala Pro Ala Pro Ala Pro Ala Glu Pro Ala Pro
    1               5                  10                  15
   Ala Pro Ala Pro Ala Gly Glu Val Ala Pro Thr Pro Thr Thr Pro Thr
               20                  25                  30
   Pro Gln Arg
           35